# EUROPÄISCHE PATENTANMELDUNG

(11) **EP 0 545 010 A1**
(43) Veröffentlichungstag der Anmeldung: **09.06.1993**
(21) Anmeldenummer: 92116112.1
(22) Anmeldetag: 21.09.1992
(51) Int. Cl.: C12Q 1/68

(54) **Verfahren zur spezifischen Vervielfältigung von Nukleinsäuresequenzen**

(30) Priorität: 26.09.1991 DE 4132132; 21.04.1992 DE 4213029
(71) Anmelder: BOEHRINGER MANNHEIM GMBH, D-68298 Mannheim (DE)
(72) Erfinder: Reischl, Udo, W-8352 Grafenau (DE); Rüger, Rüdiger, Dr. rer. nat., W-8124 Seeshaupt (DE); Kaletta, Cortina, Dr. rer. nat., W-8000 München 70 (DE); Kessler, Christoph, Dr. rer. nat., W-8201 Dorfen (DE); Kleiber, Jörg, Dr. rer. nat., W-8122 Penzberg (DE)

(57) **Zusammenfassung**

Verfahren zur spezifischen Herstellung von Nukleinsäuren beruhend auf dem Prinzip der Transkription, wobei als Promotorreagenz ein Promotoroligonukleotid und ein damit hybridisierbares templatspezifisches Oligonukleotid verwendet werden und ein Verfahren zum Nachweis von Nukleinsäure, welches auf diesem Verfahren beruht.

SEQUENZPROTOKOLL

(1) ALLGEMEINE INFORMATION:

(i) ANMELDER:
(A) NAME: Boehringer Mannheim GmbH
(B) STRASSE: Sandhoferstr. 116
(C) ORT: Mannheim
(E) LAND: DE
(F) POSTLEITZAHL: 6800
(G) TELEPHON: 06217594348

(ii) ANMELDETITEL: Verfahren zur spezifischen Vervielfaeltigung
von Nukleinsaeure- sequenzen

(iii) ANZAHL DER SEQUENZEN: 8

(iv) COMPUTER-LESBARE FORM:
(A) DATENTRÄGER: Floppy disk
(B) COMPUTER: IBM PC compatible
(C) BETRIEBSSYSTEM: PC-DOS/MS-DOS
(D) SOFTWARE: PatentIn Release #1.0, Version #1.25 (EPA)

(v) GEGENWÄRTIGE ANMELDEDATEN:
ANMELDENUMMER: EP 92116112.1

(2) INFORMATION ZU SEQ ID NO: 1:

(i) SEQUENZ CHARAKTERISTIKA:
(A) LÄNGE: 73 Basenpaare
(B) ART: Nukleinsäure
(C) STRANGFORM: Einzel
(D) TOPOLOGIE: linear

(ii) ART DES MOLEKÜLS: RNS (genomisch)

(xi) SEQUENZBESCHREIBUNG: SEQ ID NO: 1:

GCUUGAUCCG GCUACCUGCC CAUUCGACCA CCAAGCGAAA CAUCGCAUCG AGCGAGCACG 60

UACUCGGAUG GAA 73

(2) INFORMATION ZU SEQ ID NO: 2:

(i) SEQUENZ CHARAKTERISTIKA:
(A) LÄNGE: 87 Basenpaare
(B) ART: Nukleinsäure
(C) STRANGFORM: Einzel
(D) TOPOLOGIE: linear

(ii) ART DES MOLEKÜLS: cDNS

(xi) SEQUENZBESCHREIBUNG: SEQ ID NO: 2:

AGTGAGTCTT ATTAATTTTT TTTAATTAAT ACGACTCACT ATAGGCGCTC GCCGCTAGCG 60

TACGACGACT GGAGTACTGG ACATGCT 87

(2) INFORMATION ZU SEQ ID NO: 3:

(i) SEQUENZ CHARAKTERISTIKA:
(A) LÄNGE: 67 Basenpaare
(B) ART: Nukleinsäure
(C) STRANGFORM: Einzel
(D) TOPOLOGIE: linear

(ii) ART DES MOLEKÜLS: cDNS

(xi) SEQUENZBESCHREIBUNG: SEQ ID NO: 3:

TGGTCGAATG GGCAGGTAGC AGCATGTCCA GTACTCCAGT CGTCGTACGC TAGCGGCGAG 60

CGCCTAT 67

(2) INFORMATION ZU SEQ ID NO: 4:

(i) SEQUENZ CHARAKTERISTIKA:
(A) LÄNGE: 20 Basenpaare
(B) ART: Nukleinsäure
(C) STRANGFORM: Einzel
(D) TOPOLOGIE: linear

(ii) ART DES MOLEKÜLS: cDNS

(xi) SEQUENZBESCHREIBUNG: SEQ ID NO: 4:

CGATGCGATG TTTCGCTTGG 20

(2) INFORMATION ZU SEQ ID NO: 5:

(i) SEQUENZ CHARAKTERISTIKA:
(A) LÄNGE: 106 Basenpaare
(B) ART: Nukleinsäure
(C) STRANGFORM: Einzel
(D) TOPOLOGIE: linear

(ii) ART DES MOLEKÜLS: cDNS

(xi) SEQUENZBESCHREIBUNG: SEQ ID NO: 5:

GCTAGCGGCG AGCGCCTATA GTGAGTCGTA TTAATTTTTT TTAATTAATA CGACTCACTA 60

TAGGCGCTCG CCGCTAGCGT ACGACGACTG GAGTACTGGA CATGCT 106

(2) INFORMATION ZU SEQ ID NO: 6:

(i) SEQUENZ CHARAKTERISTIKA:
(A) LÄNGE: 48 Basenpaare
(B) ART: Nukleinsäure
(C) STRANGFORM: Einzel
(D) TOPOLOGIE: linear

(ii) ART DES MOLEKÜLS: cDNS

(xi) SEQUENZBESCHREIBUNG: SEQ ID NO: 6:

TGGTCGAATG GGCAGGTAGC AGCATGTCCA GTACTCCAGT CGTCGTAC 48

(2) INFORMATION ZU SEQ ID NO: 7:

(i) SEQUENZ CHARAKTERISTIKA:
(A) LÄNGE: 16 Basenpaare
(B) ART: Nukleinsäure
(C) STRANGFORM: Einzel
(D) TOPOLOGIE: linear

(ii) ART DES MOLEKÜLS: cDNS

(xi) SEQUENZBESCHREIBUNG: SEQ ID NO: 7:

GCTAGCGGCG AGCGCC 16

(2) INFORMATION ZU SEQ ID NO: 8:

(i) SEQUENZ CHARAKTERISTIKA:
(A) LÄNGE: 20 Basenpaare
(B) ART: Nukleinsäure
(C) STRANGFORM: Einzel
(D) TOPOLOGIE: linear

(ii) ART DES MOLEKÜLS: cDNS

(xi) SEQUENZBESCHREIBUNG: SEQ ID NO: 8:

CACATCATCC TGATCGACAA 20

## Beschreibung

Gegenstand der Erfindung sind ein Verfahren zur spezifischen Herstellung von Nukleinsäuren und ein Verfahren zum spezifischen Nachweis von Nukleinsäuren sowie Reagenzien zur Durchführung dieser beiden Verfahren.

Nukleinsäuren sind für alle bisher bekannten Organsimen als Informationsträger die Grundlage spezifischer Lebensformen. Sie codieren für Proteine, manche Nukleinsäuren haben jedoch wahrscheinlich auch katalytische und strukturelle Wirksamkeiten. Nukleinsäuren können wegen ihrer Spezifität auch zur Unterscheidung und zum Nachweis von Organismen herangezogen werden. Die einzelnen Nukleinsäuren sind in Organismen jedoch in nur sehr begrenzter Menge vorhanden. Zur praktischen Handhabbarkeit von Nukleinsäuren hat es sich daher als günstig erwiesen, diese Nukleinsäuren in vivo (Klonierung) oder in vitro (Amplifikation) zu vermehren. Während der erste Weg zeitraubend und umständlich ist, wurde die in vitro-Amplifikation in den letzten Jahren zu einer praktikablen Alternative entwickelt.

In der EP-A-200 362 ist ein Verfahren beschrieben, welches eine in Zyklen verlaufende Amplifikation eines Teils einer Startnukleinsäure zum Gegenstand hat. In jedem Zyklus entsteht zu den vorhandenen Nukleinsäuren jeweils ein Gegenstrang. Die Reaktionsführung hat jedoch eine relativ hohe Zyklenzahl zur Folge.

Diesen Nachteil versucht man in Verfahren, die auf Transkriptionsschritten beruhen, welche in Zyklen zu einer Vielzahl von Kopien führen, zu umgehen. Ein solches Verfahren ist beispielsweise in der EP-A-O 310 229 beschrieben. Darin wird ein Oligonukleotid (Promotorprimer), welches sowohl einen Templatespezifischen Bereich als auch eine T7-Promotorsequenz enthält, an der Templatnukleinsäure mit Mononukleotiden verlängert. Mittels eines zweiten Primers wird dann ein Gegenstrang gebildet. Dabei wird auch zu dem bislang einzelsträngigen Promotorbereich ein Gegenstrang gebildet und damit die Funktionalität des Promotors hergestellt. Anschließend findet eine Promotor-kontrollierte Transkription des gebildeten Hybrids statt. Zu den gebildeten Transkript-RNAs wird mittels eines Gegenstrangprimers cDNA hergestellt. Das Hybrid wird denaturiert und die cDNA erneut mit Promotorprimer umgesetzt. Verlängerung dieses Primers an der cDNA führt wiederum zu einem Hybrid, welches einen funktionellen Promotor enthält. Dieses Molekül kann ebenfalls in den Transkriptionszyklus eingeführt werden. Nachteilig an dieser Reaktionsführung ist die Tatsache, daß zwei Verlängerungsreaktionen zur Herstellung eines transkribierbaren Moleküls erforderlich sind. Die selben Probleme treten auch bei dem Verfahren der WO 88/10315 und der EP-A-0 329 822 sowie der EP-A-0 373 960 auf.

In der EP-A-0 427 074 wird ein Verfahren vorgeschlagen, bei dem die Templatnukleinsäure mit einem templatspezifischen promotorhaltigen Primer direkt zu einem transkribierbaren Molekül umgesetzt wird. Die anschließende Transkription ergibt RNA, welche zum einen Teil Teilsequenzen der Templatnukleinsäure entspricht und zu einem anderen Teil komplementär einer auf dem Primer befindlichen weiteren Sequenz ist. Ebenfalls darin beschrieben ist ein Verfahren, bei dem zwei verschiedene Primer benutzt werden, welche im an die Templatnukleinsäure hybridisierten Zustand ligiert werden, wobei sich ein verlängertes transkribierbares Molekül bildet. Ein Nachteil dieser Verfahren ist, daß auch bei Abwesenheit von Templatnukleinsäuren Transkriptionsprodukte des überstehenden 5'-Bereiches des Promotorprimers gebildet werden. Dies führt zu einem nur von der Menge des anwesenden Promotorprimers abhängigen Hintergrundsignal (unspezifische Amplifikation, Sensitivitätsverlust). Aus diesem Grund ist die in der EP-A-0 427 074 beschriebene Reaktion nur sehr schlecht als Startreaktion für auf Transkription beruhenden zyklischen Transkriptionsreaktionen einsetzbar. Es hat sich nämlich erwiesen, daß die Konzentration des Promotorprimers sehr viel höher als die Templatnukleinsäurekonzentration sein muß, da der Promotorprimer in jedem Zyklus verbraucht wird.

In der EP-A-0 369 775 und der EP-A-0 427 073 ist eine weitere Reaktion zur Synthese einer transkribierbaren Nukleinsäure beschrieben. Dabei wird die Templatnukleinsäure an ihrem 3'-Ende mit dem 5'-Ende eines Promotorprimers ligiert. Durch Hybridisierung mit der templatspezifischen Sequenz des überstehenden 3'-Endes wird das Templat an den Promotorprimer gebunden. Diese Reaktion benötigt daher ebenfalls eine enzymatische Reaktion zur Herstellung des transkribierbaren Moleküls.

Außerdem hat sie den Nachteil, daß nur Templatnukleinsäuren mit einem definierten 3'-Ende nachgewiesen werden können.

Aufgabe der vorliegenden Erfindung war es daher, ein auf einer Transkriptionsreaktion beruhendes Amplifikationsverfahren zur Verfügung zu stellen, welches die Nachteiles des Standes der Technik vermeidet. Insbesondere sollte es in wenigen Schritten ein Produkt liefern, welches in einen Transkriptionszyklus eingeschleust werden kann, und die bei der Transkription unter Verwendung von Promotorprimern auftretenden Sensitivitätsprobleme verringern.

Diese Aufgabe wird durch die nachfolgend beschriebene Erfindung gelöst.

Gegenstand der Erfindung ist ein Verfahren zur spezifischen Herstellung von Nukleinsäuren durch
- Umsetzung eines Promotorreagenzes P mit einer Templatnukleinsäure T unter Bildung eines transkribierbaren Nukleinsäurekomplexes K und
- promotorgesteuerte Transkription unter Bildung von Transkripten R,
wobei das Promotorreagenz P ein Promotoroligonukleotid P1 und ein damit hybridisierbares templatspezifisches Oligonukleotid P2 enthält. Ein weiterer Gegenstand ist ein Verfahren zum spezifischen Nachweis von Nukleinsäuren, welches auf dem erfindungsgemäßen Herstellungsverfahren von Nukleinsäuren beruht, und Reagenzien, die zur Durchführung der Verfahren geeignet sind.

Bei dem erfindungsgemäßen Verfahren handelt es sich um eine spezielle Ausführungsform der sogenannten Hybridisierungstests, die in ihren Grundzügen dem Fachmann auf dem Gebiet der Nukleinsäurediagnostik bekannt sind. Soweit experimentelle Details im folgenden nicht ausgeführt sind, wird dazu vollinhaltlich auf "Nucleic acid hybridisation", Herausgeber B.D. Hames und S.J. Higgins, IRL Press, 1986, insbesondere in den Kapiteln 1 (Hybridisation Strategy), 3 (Quantitative Analysis of Solution Hybridisation) und 4 (Quantitative Filter Hybridisation), Current Protocols in Molecular Biology, Edt. F.M. Ausubel et al., J. Wiley and Son, 1987, insbesondere 2.9.1. - 2.9.10 und Molecular Cloning, Edt. J. Sambrook et al., CSH, 1989, insbesondere 9.4.7. - 9.5.8. Bezug genommen. Dazu gehören insbesondere die bekannten Methoden zur Herstellung von markierten Nukleosidtriphosphaten, wie sie auch in EP-A-0 324 474 beschrieben sind, die chemische Synthese von modifizierten und unmodifizierten Oligonukleotiden, die Spaltung von Nukleinsäuren mittels Restriktionsenzymen, die Auswahl von Hybridisierungsbedingungen, durch welche eine Spezifität erreicht werden kann, die vom Ausmaß der Homologie zwischen den zu hybridisierenden Nukleinsäuren, deren GG-Gehalt und deren Länge abhängt, sowie die Bildung von Nukleinsäuren aus Nukleosidtriphosphaten mit Hilfe von Polymerasen, gegebenenfalls unter Verwendung von sogenannten Primern.

Eine Markierung im Sinne der vorliegenden Erfindung besteht aus einer direkt oder indirekt nachweisbaren Gruppe L. Direkt nachweisbare Gruppen sind beispielsweise radioaktive (³²P), farbige, oder fluoreszierende Gruppen oder Metallatome. Indirekt nachweisbare Gruppen sind beispielsweise immunologisch oder enzymatisch wirksame Verbindungen, wie Antikörper, Antigene, Haptene oder Enzyme oder enzymatisch aktive Teilenzyme. Diese werden in einer nachfolgenden Reaktion oder Reaktionssequenz detektiert. Besonders bevorzugt sind Haptene, da mit ihnen markierte Nukleosidtriphosphate (rNTP oder dNTP) im allgemeinen besonders gut als Substrate von (RNA- bzw. DNA-) Polymerasen einsetzbar sind und eine anschließende Reaktion mit einem markierten Antikörper gegen das Hapten oder das haptenisierte Nukleosid leicht vorgenommen werden kann. Solche Nukleosidtriphosphate sind beispielsweise Brom-Nukleosidtriphosphate oder Digoxigenin-, Digoxin- oder Fluorescein-gekoppelte Nukleosidtriphosphate. Als besonders geeignet haben sich die in EP-A-0 324 474 genannten Steroide und deren Detektion erwiesen. Für deren Inkorporation in Nukleinsäuren wird hiermit auf die EP-A-0 324 474 verwiesen.

Unter einem spezifischen Herstellungsverfahren oder Nachweis wird ein Verfahren verstanden, durch welches gewünschtenfalls selektiv bestimmte Nukleinsäuren auch in Gegenwart anderer Nukleinsäuren hergestellt bzw. nachgewiesen werden können. Es ist jedoch auch möglich, mehrere Nukleinsäuren oder eine Gruppe von Nukleinsäuren mit teilweise übereinstimmender oder ähnlicher Nukleotidsequenz oder mehrere Abschnitte einer Nukleinsäure in Gegenwart anderer Nukleinsäuren zum Gegenstand des Herstellungsverfahrens zu machen bzw. nachzuweisen. Zum Nachweis doppelsträngiger Nukleinsäuren kann jeder der beiden komplementären Stränge einbezogen werden.

Unter einer im wesentlichen komplementären Nukleinsäure oder Nukleinsäuresequenz werden Nukleinsäuren oder Sequenzen verstanden, die mit der entsprechenden Nukleinsäure hybridisieren können, deren Nukleotidsequenz im hybridisierenden Bereich entweder genau komplementär zu der anderen Nukleinsäure ist oder sich in wenigen Basen von der genau komplementären Nukleinsäure unterscheidet.

Die Spezifität richtet sich dabei sowohl nach dem Grad der Komplementarität als auch nach den Hybridisierungsbedingungen. Im wesentlichen zu einem Teil einer Templatnukleinsäure komplementäre Oligonukleotide werden im folgenden als templatspezifisch bezeichnet.

Grundlage der erfindungsgemäßen Verfahren sind Proben, welche Nukleinsäuren gereinigt oder in Kombination mit anderen Bestandteilen, insbesondere anderen Nukleinsäuren, enthalten. Die Probe kann weitere Bestandteile wie Proteine, Salze, etc. enthalten. Mit dem erfindungsgemäßen Verfahren ist es möglich, Nukleinsäuresequenzen zu vervielfältigen, wenn diese Sequenzen in einer in der Probe enthaltenen Nukleinsäure vorhanden sind. Die Nukleinsäure, welche der erfindungsgemäßen Herstellung von andern Nukleinsäuren zugrunde gelegt werden soll, wird im folgenden als Templatnukleinsäure (oder Template) bezeichnet.

Mit dem erfindungsgemäßen Verfahren können Nukleinsäure hergestellt werden, die die gesamte Nukleotidsequenzeninformation der Templatnukleinsäure, bevorzugt aber nur Teile davon enthalten. Zur Vermehrung von Teilsequenzen der Templatnukleinsäure ist es nicht erforderlich, jedoch möglich, die Templatnukleinsäure vor Durchführung des Verfahrens zu fragmentieren.

Die Templatnukleinsäure muß zur Durchführung des Verfahrens einzelsträngig vorliegen. Dies ist bei RNA normalerweise ohne weitere Vorbehandlung der Fall. Im Falle von DNA kann eine doppelsträngige Templatnukleinsäure durch Denaturierung auf bekannte Weise einzelsträngig gemacht werden.

Als Promotorreagenz P wird im folgenden ein Reagenz bezeichnet, das sowohl funktionelle Promotorsequenzen als auch templatspezifische Sequenzen aufweist. Die Promotoreagenzien des Standes der Technik bestanden aus einem Oligonukleotid mit einem Promotorbereich und einem templatspezifischen Bereich.

Das Promotorreagenz der Erfindung hingegen enthält ein Promotoroligonukleotid und ein damit hybridisierbares templatspezifisches Oligonukleotid.

Ein Promotoroligonukleotid P1 im Sinne der Erfindung ist eine Nukleinsäure, welche einen im wesentlichen im upstream-Bereich, bevorzugt vollständig, doppelsträngigen Bereich PRO enthält, welcher durch Erkennung und Bindung von RNA-Polymerase die Synthese von RNA startet. Dieser Bereich PRO enthält eine Sequenz, welche die Transkription des an diese Sequenz in 5'-Richtung anschließenden Nukleinsäurebereiches durch eine RNA-Polymerase initiiert. Die doppelsträngige Sequenz PRO hat vorzugsweise eine Länge von 17-100 Basen, besonders bevorzugt 17-50 Basen. Geeignete doppelsträngige Sequenzen, die eine RNA Polymerase binden kann, sind beispielsweise in Nucleic Acids Research 12, Seite 7035-7056 (1984) und in Proteinsequences and DNA Analysis 1, Seite 269-280 (1988), Biophysical Chemistry, Part III, S. 1183-1211, Freeman & Co., San Francisco, 1980; J. Bacteriol 170, S. 5248-5256 (1988); Biochem. J. 224, S. 799-815 (1984); Gene Acal. Tech. 6, S. 29-32 (1989), EP-A-0 292 802 und Nucleic acid probes, ed Symons (CRC Press, Boca Raton, 1989) beschrieben. Die beiden Stränge des doppelsträngigen Anteils können entweder offen vorliegen oder upstream in Form einer Haarnadelstruktur (Loop) verbunden sein. Der die komplementären Einzelstränge verbindende Haarnadelbereich ist vorzugsweise 5-50 Nukleotide, besonders bevorzugt 5-10 Nukleotide lang und ist zur Vermeidung der Ausbildung von Basenpaaren bevorzugt aus nur einer Nukleotidart aufgebaut. Am downstream 3'-Ende weist das Promotoroligonukleotid P1 einen einzelsträngigen Bereich OBS auf. Dieser Bereich ist zu der Templatnukleinsäure und zu anderen in der Probe enthaltenen, nicht erfindungsgemäß zu verarbeitenden Nukleinsäuren garnicht oder nur begrenzt komplementär, so daß er nicht direkt mit diesen Nukleinsäuren hybridisieren kann. Der Bereich OBS ist vorzugsweise 6 bis 50 nt, besonders bevorzugt 12 bis 30 nt lang.

Der doppelsträngige Promotorbereich PRO kann von der Oligonukleotid-Bindungsstelle OBS durch eine Sequenz von weiteren Nukleotiden getrennt sein. Außerdem kann sich an den Bereich OBS in downstream 3'-Richtung eine weitere Nukleotidsequenz anschließen.

Das 3'-Ende des Promotoroligonukleotids P1 ist bevorzugt nichtphosphoryliert, während das 5'-Ende phosphoryliert oder nichtphosphoryliert sein kann. Das Promotoroligonukleotid P1 kann außerdem in downstream Richtung anschließend an die Sequenz PRO eine weitere Nukleotidsequenz enthalten. Bevorzugt sind dies Nukleotidsequenzen, welche zu dem entsprechenden Gegenstrang komplementär sind. Bevorzugt sind transkriptionsfördernde Sequenzen (Nucl. Acids Res. 15, S. 8783-8798 (1987)). Diese sind bevorzugt 1-10 nt. Es können aber auch Nukleotide sein, die zusätzliche Reaktionsschritte ermöglichen (z.B. ori-Sequenzen, RNA-Replikaseerkennungsstelle, Restriktionsschnittstellen).

Das templatspezifische Oligonukleotid P2 enthält eine einzelsträngige Nukleotidsequenz TEM 1', welche im wesentlichen komplementär zu einer Nukleotidsequenz der Templatnukleinsäure und daher mit dieser hybridisierbar ist. Diese Sequenz ist bevorzugt 6 bis 50 nt, besonders bevorzugt 12 bis 30 nt lang. Durch ihre Länge und Komplementarität läßt sich die Spezifität des erfindungsgemäßen Herstellungsverfahrens steuern. Durch geeignete Wahl dieser Sequenz ist es beispielsweis möglich, spezifisch nur eine oder auch mehrere Templatnukleinsäuren der Probe zum Gegenstand des erfindungsgemäßen Verfahren zu machen und ihre Sequenz zu vermehren.

Das templatspezifische Oligonukleotid P2 enthält außerdem eine Nukleotidsequenz OBS', welche spezifisch mit der Sequenz OBS des Promotors P1 hybridisieren kann. Bevorzugt ist diese Sequenz OBS' im wesentlichen komplementär zu der Sequenz OBS. Die Sequenz TEM 1', liegt bevorzugt im 5'-Endbereich des Oligonukleotids P2 während die Sequenz OBS' bevorzugt im 3'-Endbereich von P2 liegt. Dazwischen können zusätzliche Sequenzen liegen. P2 kann upstream von OBS' Nukleotidsequenzen enthalten, die einem Teil von PRO entsprechen. Ein wesentliches Merkmal der Erfindung ist die Tatsache, daß das 3'-Ende des Oligonukleotids P2 nicht kovalent mit dem 5'-Ende des Promotoroligonukleotids P1 verbunden ist. Unter Hybridisierungsbedingungen für die Sequenz OBS/OBS' befindet sich zwischen dem 3'-Ende von P2 und dem darauf zuzeigenden 5'-Ende des P1 keine kovalente Bindung. An dieser Stelle kann beispielsweise ein Nick sein. Der Nick kann an der Position -5 bis +30 liegen, bezogen auf die Initiationsstelle der Transkription. Bevorzugt liegt der Nick im Bereich -5 bis -1 oder +15 bis +30, wenn die Initiationsstelle als Position +1 bezeichnet ist. OBS ist bevorzugt 6-50 nt, besonders bevorzugt 12-30 nt, lang.

In einem ersten Schritt des erfindungsgemäßen Verfahrens zur spezifischen Herstellung von Nukleinsäuren wird die Probe, welche die Templatnukleinsäure enthält, mit dem Promotorreagenz P unter Hybridisierungsbedingungen umgesetzt. Dabei bildet sich ein transkribierbarer Nukleinsäurekomplex K aus, bei welchem die Templatnukleinsäure über einen doppelsträngigen Bereich TEM 1/TEM 1' an das Oligonukleotid P2 und dieses wiederum über einen doppelsträngigen Bereich OBS/OBS' an den Promotor hybridisiert ist.

Die Spezifität und Sensitivität des erfindungsgemäßen Verfahrens kann durch den Einsatz eines weiteren Oligonukleotids P3 erheblich gesteigert werden. Es wird bevorzugt gleichzeitig mit dem Promotorreagenz eingesetzt und bildet zusammen mit diesem und T den Transkriptionskomplex K. Es ist prinzipiell möglich, in 3'-Richtung der Templatnukleinsäure noch weitere Oligonukleotide anzuhybridisieren, die dieselbe Funktion wie P3 haben. Primer P3 hybridisiert an eine in 3'-Richtung der Templatnukleinsäure liegende Nukleotidsequenz TEM 2 der Templatnukleinsäure. Primer P3 weist dazu im 3'-Bereich eine einzelsträngige Nukleotidsequenz TEM 2' auf, welche im wesentlichen komplementär zu der TEM 2 ist. Der Bereich TEM 1 ist von dem Bereich TEM 2 der Nukleinsäure verschieden. Bevorzugt liegen die Bereiche TEM 1 und TEM 2 direkt nebeneinander auf der Templatnukleinsäure, so daß sie nur durch ein nick getrennt sind. Sie können jedoch auch 1-1500, bevorzugt 1-150 nt voneinander entfernt sein. Diese Lücke (a) kann dann bevorzugt vor Start der Transkription durch die Aktivität einer DNA-abhängigen DNA-Polymerase oder eine RNA-abhängigen DNA-Polymerase, wie z.B. reverse Transkriptase, geschlossen werden. Die Lücke muß durch diese Elongationsaktivität nicht komplett geschlossen sein; kurze Lücken von 1-5 Nukleotiden zwischen dem 5'-Ende von P2 und dem 3'-Ende von P3 führen trotzdem zur Bildung eines Transkriptions-Produkts, welches die Sequenzen aus P2, der elongierten Sequenz (a') und P3 enthält. Im hybridisierten Zustand sind das 5'-Ende des Oligonukleotids P2 und das 3'-Ende des Primers P3 bzw. das elongierte Ende einander direkt benachbart, nicht jedoch miteinander kovalent verbunden. Der Primer P3 kann über die Sequenz TEM 2' hinaus noch weitere Nukleotide enthalten, die nicht mit der Templatnukleinsäure hybridisieren. Eine solche Sequenz könnte z. B. eine ORI-Sequenz (Startregion für eine Replikation) eine RE-Schnittstelle, eine replikasespezifische Sequenz oder eine Binderegion für einen Sequenzierprimer oder ein Bindeprotein sein. P3 hat am 5'- und 3'-Ende bevorzugt eine OH-Gruppe. Die Hybridisierungsbereiche TEM 1, TEM 2 sowie die später beschriebenen TEM 3 und TEM 4 sind bevorzugt 6-50 nt, besonders bevorzugt 12-30 nt lang.

Ein wesentlicher Vorteil der Erfindung ist, daß die Menge des Promotoroligonukleotids P1 unabhängig von der Menge an templatspezifischen Sequenzen (Oligonukleotid P2) gehalten werden kann. Eine wesentliche Erkenntnis der Erfindung ist nämlich, daß die Sensitivität bei später durchgeführten zyklischen Transkriptionsreaktionen gegenüber dem Stand der Technik erhöht werden kann, wenn die Menge an templatspezifischen Sequenzen im Verhältnis zu Promotorsequenzen nur relativ gering ist. Bevorzugt beträgt die Stöchiometrie des Einsatzes von P1 zu P2 100:1 bis 2:1, besonders bevorzugt 50:1 bis 5:1. Die Konzentration des Promotoroligonukleotids P1 richtet sich dann im wesentlichen danach, wie groß die Anzahl der herzustellenden Nukleinsäuren sein soll. Auch das Mengenverhältnis von Primer P3 zu P2 wird hoch gewählt, bevorzugt 100:1 bis 2:1, besonders bevorzugt 50:1 bis 5:1. Die Konzentration an P3 kann sich in erster Linie an der Konzentration von P1 orientieren. Bevorzugt sind die Konzentrationen von P1 und P3 in etwa gleich.

Der somit gebildete Nukleinsäurekomplex K wird nun einer promotorgesteuerten Transkription unterworfen. Die Bedingungen, unter denen eine promotorgesteuerte Transkription ablaufen kann, sind für diesen Komplex die gleichen wie für die Transkriptionsreaktionen des Standes der Technik (siehe z.B. EP-A-0 369 775). Sie hängen von dem gewählten Promotor/Polymerase-System ab. Beispiele für Promotorsysteme sind aus T7, SP6, N4 und T3 bekannt. (Für N4 siehe RNA-Polymerase and the Regulation of Transcription, Ed. Reznikoff, Elsevier Science, 1987, Seite 37-45). Im wesentlichen sind eine RNA-Polymerase und Ribonukleosidtriphosphate (rNTPs) erforderlich. Als besonders bevorzugtes Transkriptionssystem hat sich das Transkriptionssystem von T7 (T7-RNA-Polymerase und T7-spezifischem Promotor) bewährt.

Die T7-RNA-Polymerase-promotor-spezifischen komplementären Sequenzbereiche innerhalb der doppelsträngigen Region des Primers P1 können zwischen 12 und 20 Nukleotide lang sein (Milligan et al. (1987), Nucl. Acids Res., 15, S. 8783-8798 beschrieben ist). Bevorzugt liegt er aber in einer Länge von 17 Nukleotiden vor. Beispielsweise beginnt die Transkription durch T7-RNA-Polymerase in Position +1 (Abb. auf Seite 2690 in Biochemistry 1987, 26, 2690-2696). Bevorzugt wird die Transkription initiert, wenn +1 und +2 in der Sequenz des Primers P1 C darstellen. Weitere Details sind in Gentechnologie von A-Z, Seiten 222-223 (Verlag VCH, Weinheim 1990, Stichwort "Gen") beschrieben. Ferner wird auf Proc. Natl. Acad. Sci. USA 83, 3614 (1986) und Gene 72, S. 75 (1989) hingewiesen. Auf den Inhalt dieser Publikationen wird ausdrücklich verwiesen.

Eine wesentliche Erkenntnis für das erfindungsgemäße Verfahren ist, daß die promotorgesteuerte Transkription auch solcher doppelsträngiger Nukleinsäuren möglich ist, die im doppelsträngigen Bereich Einzelstrangbrüche aufweisen. Es war weiter erstaunlich, daß diese Einzelstrangbrüche auch innerhalb der Promotorsequenz liegen können.

Produkt der Transkription sind RNA-Transkripte. Hauptprodukt ist eine RNA R, deren 5'-Ende beim Transkriptionsstartpunkt und deren 3'-Ende in der 5'-Endposition des Bereiches TEM 2' liegt. Diese RNA enthält insbesondere die Sequenz OBS sowie Sequenzen, die homolog zu TEM 1 und TEM 2 sind.

Das Produkt der nicht spezifischen Transkription des Hybridisierungsprodukts aus P1 und P2, welches einen ebenfalls transkribierbaren Nukleinsäurekomplex K' darstellt, entsteht bei dem erfindungsgemäßen Verfahren nur in vergleichsweise geringen Mengen, da die Menge dieses Komplexes wegen der geringen anwesenden Menge an Oligonukleotid P2 ebenfalls gering ist. Dieses kurze Transkript würde unter Umständen spätere Transkriptionsreaktionen stören.

Das als Hauptprodukt entstehende Transkript R kann das Endprodukt des erfindungsgemäßen Verfahrens zur Herstellung von Nukleinsäuren sein. Zweck des Herstellungsverfahrens kann dann beispielsweise die Erhöhung der Anzahl von Nukleinsäuren mit gleicher Sequenzinformation vor einer nachfolgenden Klonierung oder, bei Einbau von nachweisbar markierten Mononukleotiden, die Herstellung von nachweisbaren Nukleinsäuresonden (definierter Länge) zur Durchführung von Hybridisierungsassays sein.

Bevorzugt wird dieses Transkript jedoch in einem zyklisch verlaufenden Reaktionsprozeß weiter amplifiziert. Die dazu erforderlichen Reaktionsschritte sind prinzipiell aus dem Stand der Technik bekannt. Die im folgenden beschriebene Reaktionsfolge hat sich jedoch als besonders bevorzugt erwiesen.

Das Reaktionsgemisch aus der ersten Transkriptionsstufe wird dazu zunächst Bedingungen unterworfen, bei denen zu dem Hauptprodukt der Transkription eine cDNA (Gegenstrang G) gebildet wird. Dies kann auf einfache und bevorzugte Weise durch Zugabe von Reverser Transkriptase und Desoxyribonukleotiden (dNTPs) geschehen, wenn P3, welcher zu TEM 2 auf der RNA komplementär ist, bereits in ausreichenden Konzentrationen im Reaktionsgemisch enthalten ist. P3 wirkt dann als Primer, der enzymatisch unter Verwendung von R als Template zu G verlängert wird. Da P3 nur mit den Transkripten R hybridisieren kann, die aus einem Transkriptionskomplex K entstanden sind, nicht jedoch mit den Transkripten, die aus templatunspezifischen Transkriptionskomplexen, z.B. nur aus P1 und P2, als Nebenprodukt enstehen könnten, führt der Einsatz von P3 zu einer erheblichen Selektivitätssteigerung. Es ist ferner möglich, ein oder mehrere weitere Oligonukleotide P7...PN einzusetzen, die in Art von P3 downstream von und anschließend an P3 an die Templatnukleinsäure hybridisieren können; auch diese Oligonukleotide können als Primer für die Gegenstrangsynthese oder/und als Fang- oder Nachweissonde dienen.

In einer bevorzugten Ausführungsform können auch modifizierte Deoxyribonukleotide beispielsweise Digoxigenin-dUTP in die cDNA eingebaut werden.

In einem weiteren Schritt wird enzymatisch der RNA-Teil des gebildeten RNA/DNA-Doppelstranges z.B. durch RNAse H verdaut. Die mit Hilfe dieser RNA als Templat synthetisierte cDNA bleibt erhalten.

Diese cDNA hat in ihrem 3'-Endbereich wiederum die Sequenz OBS'. Daher kann unter hybridisierenden Bedingungen Promotor-oligonukleotid P1 direkt mit dieser cDNA über OBS unter Bildung eines transkribierbaren Nukleinsäurekomplexes L hybridisieren. Es ist hier zu bemerken, daß zur Bildung dieses transkribierbaren Komplexes das limitiert enthaltene Oligonukleotid P2 nicht mehr erforderlich ist.

Wegen des Mengeverhältnisses P1/P2 konkurriert Oligonukleotid P2 auch nur noch in untergeordnetem Maße mit der cDNA um die Bindung an das Promotoroligonukleotid P1. Die Ausbeuten an transkribierbarem Produkt L sind daher vergleichsweise hoch.

In einem weiteren Schritt findet nun die promotorgesteuerte Transkription an dem Nukleinsäurekomplex L statt. Es ist dazu nicht erforderlich, erneut Reagenzien zuzugeben, da diese bereits in dem Reaktionsgemisch aus der Startreaktion vorhanden sind. Die Zugabe von Reagenzien beispielsweise bei Aufbrauchen des Reagenzes ist jedoch prinzipiell möglich. Resultat der Transkription ist eine Vielzahl von RNA-Transkripten R, welche für die erneute cDNA-Bildung zur Verfügung stehen.

Der genannte Zyklus kann so lange fortgeführt werden, bis die gewünschte Anzahl von Nukleinsäuren gebildet ist.

Produkt des erfindungsgemäßen Herstellungsverfahrens kann jedes der in dem Zyklus gebildeten Zwischenprodukte sein, d.h. die Transkripte R, das Hybrid aus R und der cDNA, die cDNA selbst, das Hybrid aus P1 und der cDNA oder das Gemisch selbst. In einer bevorzugten Ausführungsform können auch modifizierte Ribonukleotide, z.B. Digoxigenin-UTP in das Zwischenprodukt R eingebaut werden.

Die Produkte können auf bekannte Weise gereinigt oder/und weiterverarbeitet werden.

Der Nachweis, ob eine ausreichende Zahl von Nukleinsäuren gebildet wurde, kann beispielsweise durch Zugabe einer nachweisbar markierten Detektorsonde, welche mit dem gewünschten Produkt hybridisieren kann, und Nachweis des Hybrids, oder durch direkten Nachweis der durch Einbau von nachweisbar markierten Mononukleotiden gebildeten Nukleinsäuren geschehen.

Ein Vorteil des erfindungsgemäßen Verfahrens ist, daß es isotherm geführt, d.h. bei einer Temperatur durchgeführt werden kann. Ein weiterer Vorteil ist, daß eine mögliche Nebenreaktion durch Bindung des Hybrids aus Promotoroligonukleotid P1 und Oligonukleotid P2 mit den neu entstandenen templatspezifischen Transkripten R durch Komplettierung des partiellen Doppelstranges mit Reverser Transkriptase gehemmt wird. Ebenfalls ein Vorteil des erfindungsgemäßen Verfahrens ist, daß verschiedene Nukleinsäuren bei Verwendung nur eines einzigen Promotoroligonukleotids P1 nachgewiesen werden können, da die templatspezifische Sequenz auf dem davon getrennten Oligonukleotid P2 liegt und somit unabhängig variiert werden kann (bei gleichbleibendem OBS).

In einer Ausführungsform des erfindungsgemäßen Verfahrens werden zur Stabilisierung der Einzelstrangkonfiguration der Templatnukleinsäure im Bereich der Sequenzen TEM 1 und TEM 2 blockierende Oligonukleotide P4 und P5 in den zu TEM 1 und TEM 2 benachbarten Bereichen hybridisiert werden. Der Bereich TEM 3 ist bevorzugt 2-50, besonders bevorzugt 3-10, Nukleotide von TEM 1 entfernt lokalisiert. Der Bereich TEM 4 sollte 2-50, bevorzugt 3-10 Nukleotide von der Bindungsstelle TEM 2 entfernt sein. Bevorzugt enthalten die Oligonukleotide P4 und P5 eine Modifikation, beispielsweise Dideoxyribonukleotide, zur Blockierung von Polyermaseaktivität am 3'-Ende, um Elongation von P4 oder P5 zu verhindern. Die Oligonukleotide P4 und P5 sind bevorzugt 6-50, besonders bevorzugt 12-30 nt lang.

In einer weiteren Ausführungsform liegt TEM 2 1-1500, bevorzugt 1-150 nt vom 5'-Ende des Bereiches TEM 1 entfernt. Die Lücke a zwischen P2 und P3 kann durch eine RNA-abhängige oder DNA-abhängige DNA-Polymerase (fakultativ mit RNAse H-Aktivität) und dNTP im Ansatz aufgefüllt werden. In einer weiteren Ausführungsform enthalten P1 und P2 Phosphatreste am 5'-Ende und werden nach Bildung des transkribierbaren Komplexes K miteinander oder/und mit P3 durch eine Ligasereaktion (z. B. mit T4-Ligase, E.coli-Ligase oder thermostabilen Ligasen) verbunden.

In einer weiteren Ausführungsform ragt das 5'-Ende von P1 um eine Sequenz X über den Transkriptionsstartpunkt hinaus. In diesem Fall wird bevorzugt ein weiteres Oligonukleotid P6 eingesetzt, welches solang ist wie X, bevorzugt 10-40 nt, besonders bevorzugt 15-25 nt. P6 bewirkt, daß beim RNAseverdau der X entsprechende RNA-Bereich verdaut und für X keine cDNA gebildet wird, so daß die cDNA direkt angrenzend an das 5'-Ende von P1 mit P1 hybridisieren kann. Es befindet sich also ein nick zwischen beiden Oligonukleotiden, aber kein 3'-Überhang der cDNA.

Ein weiterer Gegenstand der Erfindung ist ein Verfahren zum spezifischen Nachweis von Nukleinsäure, welches das erfindungsgemäße Verfahren zur spezifischen Herstellung von Nukleinsäuren und seine Ausführungsformen einschließt, wobei die Transkripte R oder deren Folgeprodukte als Maß für die Menge oder Anwesenheit der Templatnukleinsäure in der Probe nachgewiesen werden. Unter Folgeprodukte sind hierbei insbesondere die Zwischenprodukte der zyklischen Transkriptionsreaktion, wie z. B. die cDNA, zu verstehen.

Der Nachweis dieser Produkte kann prinzipiell auf bekannte Art und Weise geschehen, beispielsweise durch Hybridisierung mit markierten Sondennukleinsäuren und Nachweis markierter Hybride. Eine weitere einfache Methode ist der Einbau markierter Mononukleotide während der Transkriptionsreaktion und die Auftrennung der Reaktionsprodukte in der Gelelektrophorese. Dies wird besonders dadurch begünstigt, daß die gebildeten Produkte eine einheitliche Länge aufweisen.

Eine besonders bevorzugte Ausführungsform des erfindungsgemäßen Verfahrens benutzt den Einbau eines detektierbar markierten Mononukleotids während der Transkriptionsfolge (d.h. in die Transkripte oder/und deren Folgeprodukte) und die anschließende Hybridisierung mit einer Fangsonde, die entweder direkt an eine feste Phase gebunden ist oder bevorzugt durch Kopplung an eine chemische Gruppe wie z. B. Biotin immobilisierbar ist. Im Falle von immobilisierbaren Sonden ist eine anschließende Immobilisierung an eine Festphase, welche eine Bindeaffinität für die chemische Gruppe aufweist möglich. Bevorzugt wird nach Abtrennung der detektierbar markierten Mononukleotide die Markierung an der Festphase nachgewiesen. Als detektierbare Gruppe wird bevorzugt Digoxigenin (EP-A-0 324 474) verwendet. Die Anwesenheit dieser Gruppe an der Festphase wird dann über einen enzymmarkierten Antikörper gegen Digoxigenin nachgewiesen.

Als Fang- oder Nachweissonde werden bevorzugt Sequenzen gewählt, welche komplementär oder homolog zu Teilsequenzen oder der Gesamtsequenz von P3 (oder gegebenenfalls P7...PN) oder komplementär oder homolog zum 5'-Bereich von P2 und 3'-Bereich von P3 (bevorzugt jeweils 6-25 nt, besonders bevorzugt 8-13 nt) sind. Die gesamte Sonde ist 6-5000 nt, bevorzugt 12-50, besonders bevorzugt 16-26 nt lang. Bei der Auffüllreaktion (Beispiel 7 ) liegen diese Sequenzen besonders bevorzugt im Bereich zwischen P2 und P3 (Auffüllbereich); daraus ergibt sich ein Vorteil für die Spezifität der Detektion.

In einer weiteren Ausführungsform ist P3 (oder gewünschtenfalls P7...PN) mit einer zur Immobilisierung befähigten Gruppe markiert, bevorzugt am 5'-Ende. Über diese Gruppe kann die cDNA an eine Festphase gebunden werden. Mit diesem zur Immobilisierung befähigten P3 kann auch der Komplex K vor der Transkriptionsreaktion an eine Oberfläche gebunden werden. Damit wird durch einen nachfolgenden Waschschritt eine besonders gute Abreinigung unspezifischer Nukleinsäuren erreicht. In diesem Fall wird nach Wandbindung die Transkriptionsreaktion durch Zugabe der Enzyme und der Oligonukleotide P1 und P3 gestartet. Die gebundene cDNA kann entweder mittels einer markierten Detektorsonde oder durch Einbau von nachweisbar markierten Mononukleotiden während der Transkription detektiert werden. Die cDNA, welche mit der immobilisierbaren Gruppe an die Festphase gekoppelt ist, kann in einer alternativen Ausführungsform auch durch Waschen von allen unspezifischen Transkriptionsprodukten gereinigt werden. Danach wird durch erneute Zugabe von Enzymen/P1 und nicht immobilisierbarem P3 die Amplifikationsreaktion gestartet.

In einer weiteren Ausführungsform wird zuerst das Hybrid aus Templatnukleinsäure und immobilisierbarem P3 an eine Festphase gebunden. Anschließend wird nach einem Waschschritt P1 und P2 zugefügt. Nach Hybridisierung von P1 und P2 an das P3/Templat-Hybrid wird erneut gewaschen und durch Zugabe von RNA-Polymerase, P1 und nicht immobilisierbarem P3 der immobilisierte Komplex K transkribiert. Zur weiteren Reaktion wird wie oben beschrieben vorgegangen.

In einer weiteren Ausführungsform ist P1 immobilisierbar modifiziert. Dann kann beispielsweise der Transkriptionskomplex K oder/und L an eine Festphase gebunden und, z.B. über in G eingebaute markierte Mononukleotide, dessen Menge als Maß für die Anwesenheit der Templatnukleinsäure bestimmt werden.

Das erfindungsgemäße Nachweisverfahren hat alle Vorteile des erfindungsgemäßen Herstellungsverfahrens von Nukleinsäuren.

In den Figuren sind jeweils Nachweisverfahren beispielhaft gezeigt. Es handelt sich hier jedoch auch um erfindungsgemäße Herstellverfahren, wenn die Nachweisschritte weggelassen werden.

Ebenfalls Gegenstand der Erfindung sind Reagenzien und Reagenzkits zur Durchführung der erfindungsgemäßen Verfahren.

Gegenstand der Erfindung ist ein Reagenz, welches folgende Bestandteile enthält:
- ein Promotoroligonukleotid P1, welches außer der doppelsträngigen Promotorsequenz PRO eine einzelsträngige Oligonukleotidbindungsstelle OBS enthält,
- mindestens ein templatspezifisches Oligonukleotid P2, welches neben der spezifischen Sequenz eine zu OBS im wesentlichen komplementäre Nukleotidsequenz enthält.

Bevorzugt enthält das Reagenz außerdem mindestens eines der oben beschriebenen Oligonukleotide P3, P4, P5 und P6, besonders bevorzugt jedoch mindestens P3. Für die bevorzugten Mengenverhältnisse der Oligonukleotide wird auf die Angaben bei den erfindungsgemäßen Verfahren verwiesen. Ebenfalls bevorzugt enthält es die vier Mononukleosidtriphosphatarten, unmodifiziert oder nachweisbar oder immobilisierbar modifiziert. Ferner kann es pH-Puffer und Hilfsstoffe, z.B. Enzyme oder Stabilisatoren, enthalten, insbesondere solche, die für anschließende Transkriptionsreaktionen geeignet sind.

Als Stabilisatoren können beispielsweise auch doppelstrangstabilisierende Agenzien dienen, bevorzugt eine Ligase, besonders bevorzugt T4-Ligase. Durch diese Agenzien wird eine deutliche Effizienzsteigerung der Transcription erreicht, selbst wenn unter den Reaktionsbedingungen keine Ligation der Oligonukleotide miteinander möglich ist, z.B. wenn die 5'-Enden der Oligonukleotide (beispielsweise P1, P2 oder/und P3) nicht phosphoryliert sind.

Weiterer Gegenstand der Erfindung ist ein Reagenzkit, welches in getrennten Behältern enthält:
1) das Promotoroligonukleotid P1, das templatspezifische Oligonukleotid P2 und Monoribonukleosidtriphosphate;
2) ein Transkriptionsenzym, passend zum Promotor;
3) eine reverse Transkriptase; und
4) ein RNA verdauendes Enzym.

Das Reagenzkit kann die unter 1) genannten Bestandteile auch in getrennter Form enthalten. Bevorzugt enthält Behälter 1 auch schon das Oligonukleotid P3 und gewünschtenfalls die Monodesoxyribonukleosidtriphosphate. Sie können aber auch in Behälter 3 oder in einem eigenen Behälter enthalten sein.

Das Reagenzkit kann ferner Kontrollnukleinsäuren und/oder Reagenzien zur Probenvorbereitung enthalten.

Wenn das Reagenzkit zum Nachweis von Nukleinsäuren oder Nukleotidsequenzen verwendet werden soll, enthält es bevorzugt die dazu erforderlichen Reagenzien in einem getrennten Behälter, z.B. Fang- oder/und Nachweissonden.

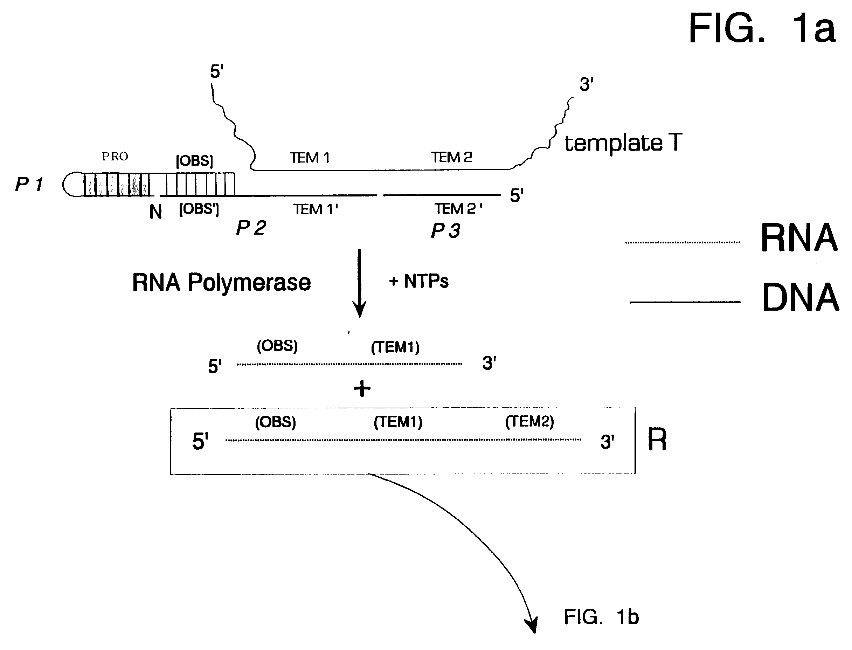

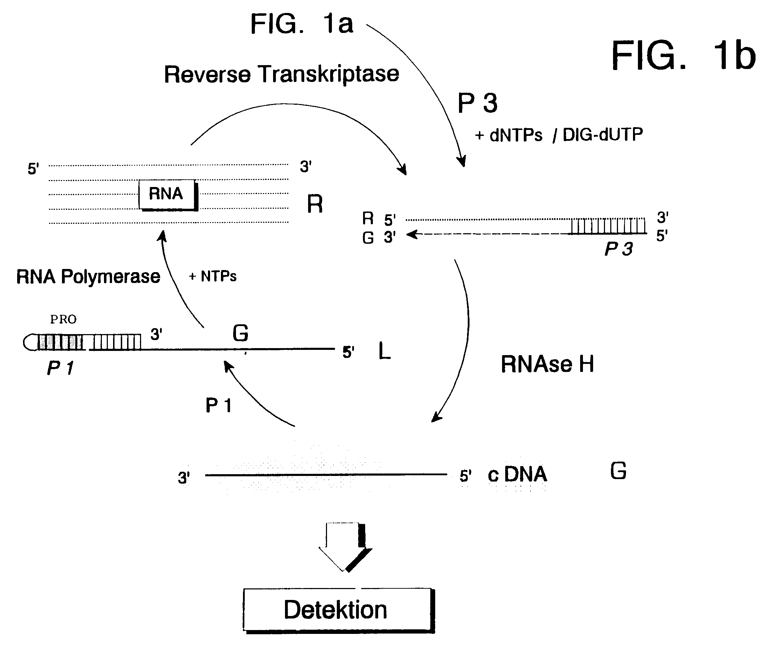

In Figur 1 (umfassend FIG 1a und FIG 1b) ist das erfindungsgemäße Nachweisverfahren schematisch in Form der bevorzugten Ausführungsform bezüglich der Verwendung eines Oligonukleotids P3 wiedergegeben.

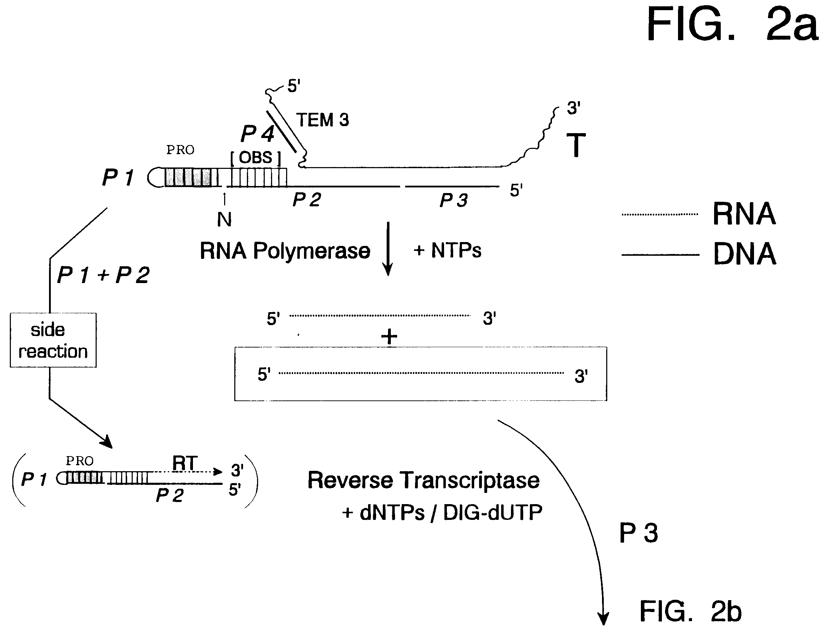

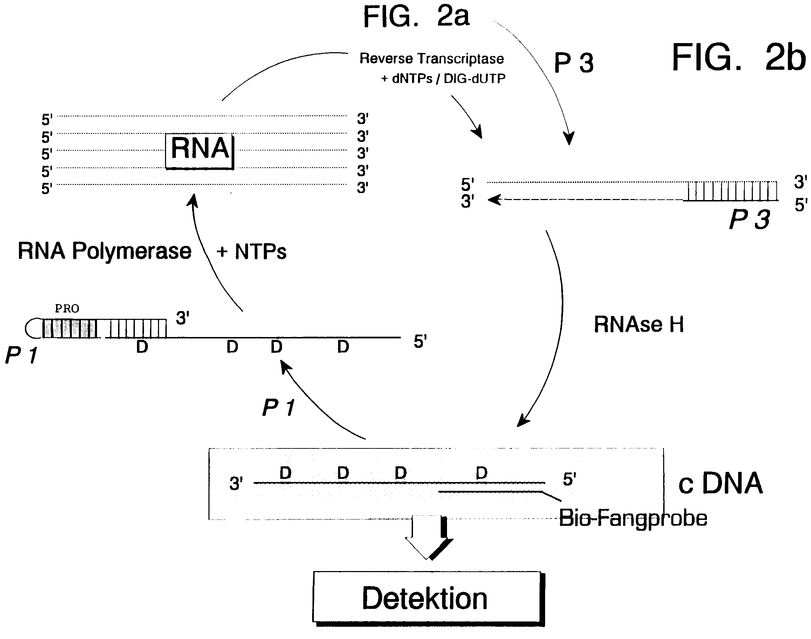

In Figur 2 (umfassend FIG 2a und FIG 2b) ist ein Verfahren wiedergegeben, bei dem auch ein einzelstrangstabilisierendes Oligonukleotid P4 eingesetzt wird. Im linken Teil dieser Figur ist außerdem die Nebenreaktion durch Reverse Transkriptase dargestellt. Die Transkriptionszyklen werden in Figur 2 beispielhaft unter Einbau von nachweisbaren markierten Monodesoxyribonukleosidtriphosphaten (z.B. Digoxigenin-dUTP) gezeigt. Es ist außerdem gezeigt, daß die markierte cDNA durch anschließende Hybridisierung mit einer biotinylierten Fangsonde und Bindung des gebildeten Hybrids an eine Streptavidin-Festphase nachgewiesen werden kann.

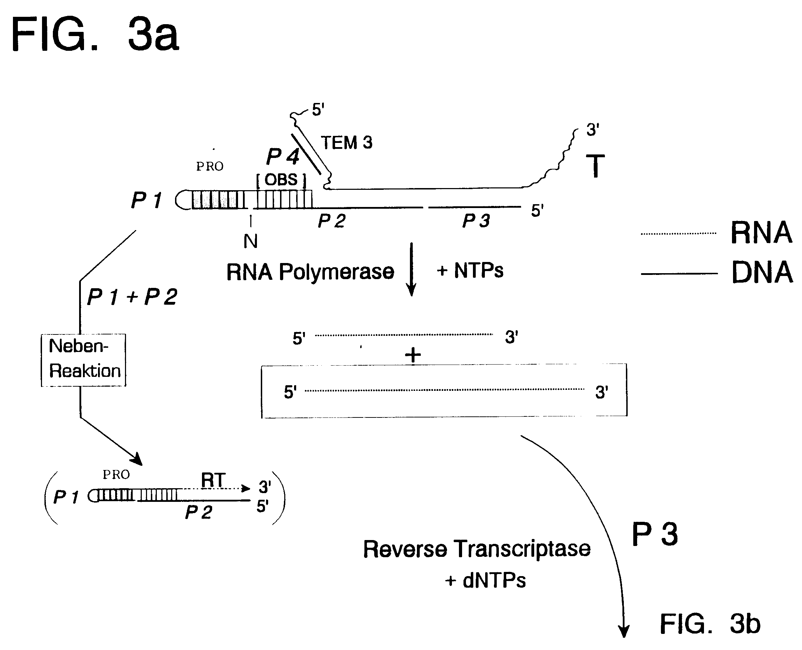

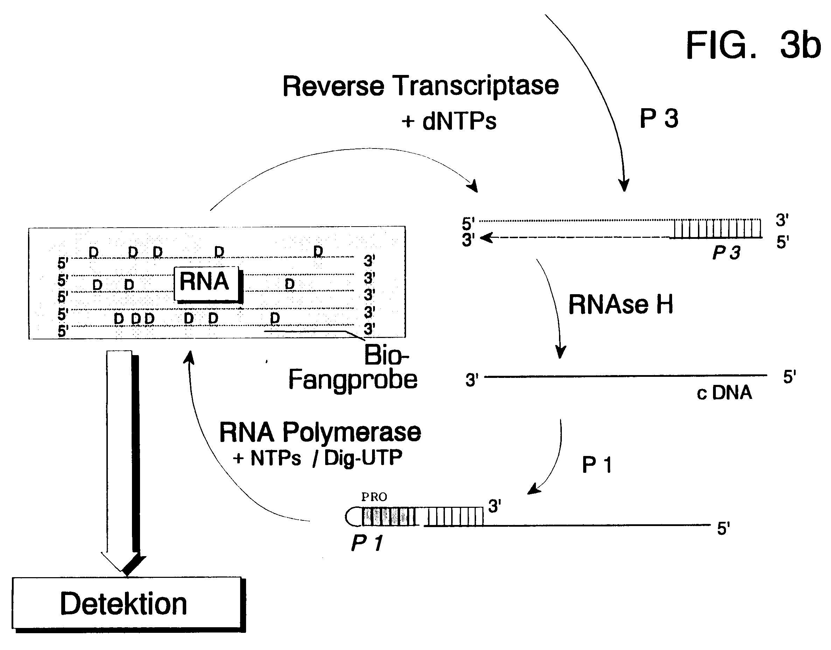

Figur 3 (umfassend FIG 3a und FIG 3b) zeigt eine Ausführungsform des erfindungsgemäßen Nachweisverfahrens unter Einbau von nachweisbar markiertem Monoribonukleosidtriphosphaten (z.B. Digoxigenin-UTP) und Abfangen der Transkripte R mittels einer biotinylierten Fangsonde.

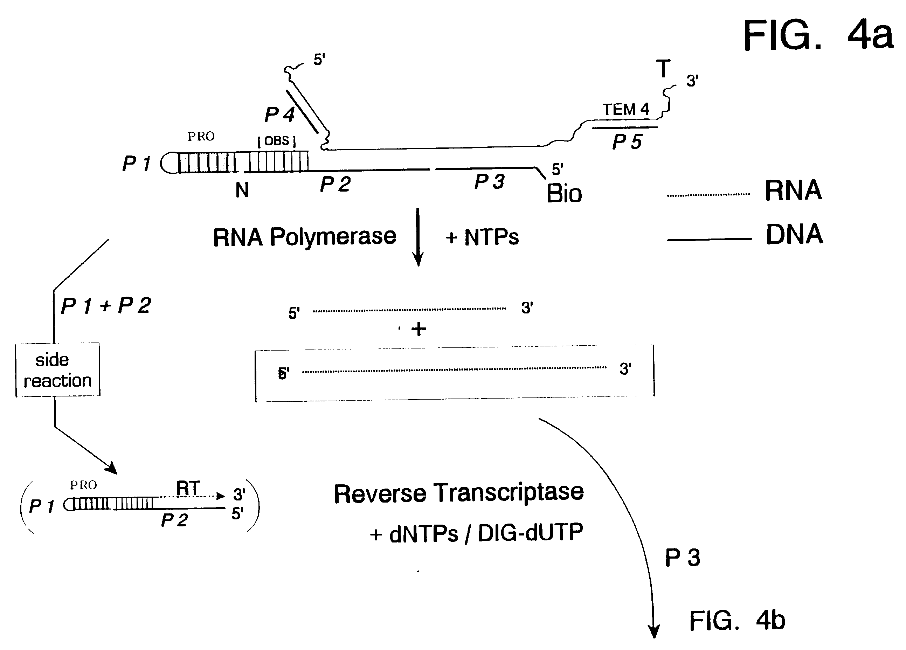

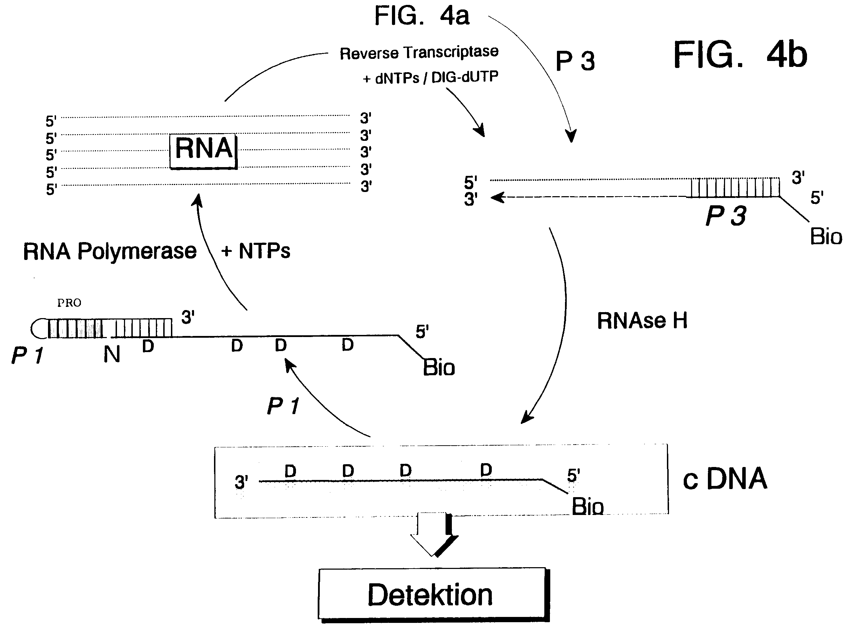

Figur 4 (umfassend FIG 4a und FIG 4b) zeigt eine Ausführungsform, bei der sowohl die einzelstrangstabilisierenden Oligonukleotide P4 und P5, als auch ein immobilisierbar substituiertes Oligonukleotid P3 verwendet werden. In dieser Ausführungsform wird die cDNA nachgewiesen, welche sowohl nachweisbar als auch immobilisierbar markiert ist.

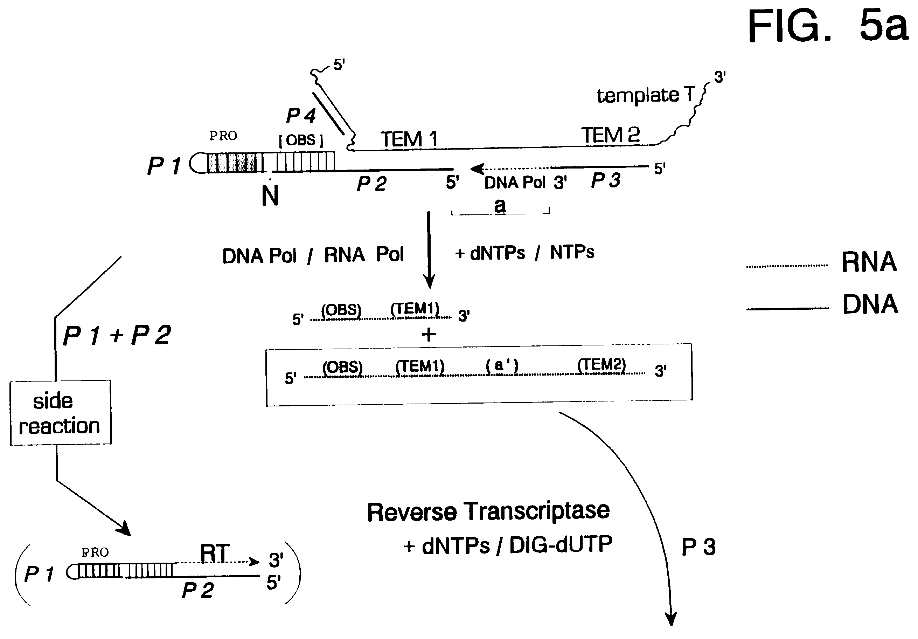

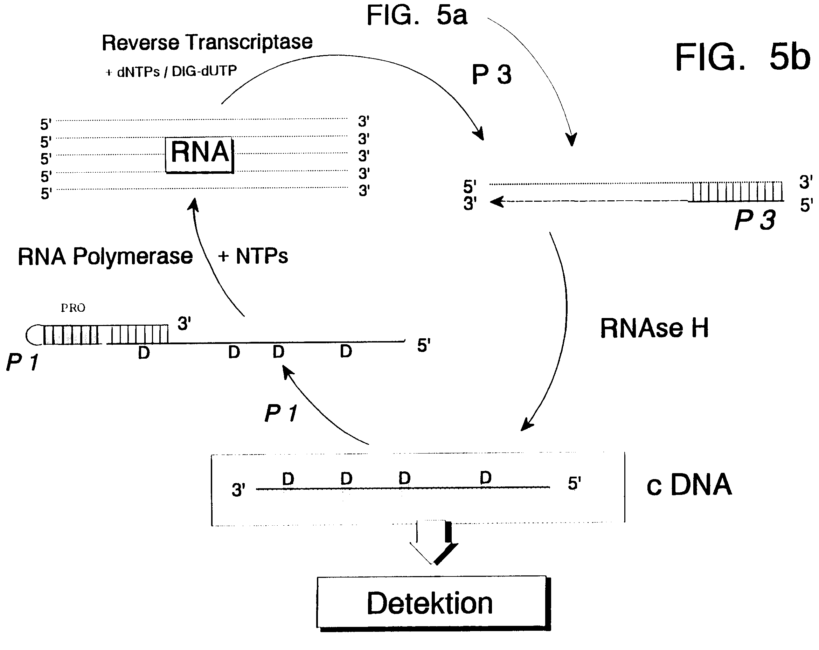

In Figur 5 (umfassend FIG 5a und FIG 5b) wird eine Ausführungsform gezeigt, bei der nach Bildung des Nukleinsäurekomplexes K zunächst mittels DNA-Polymerase ein einzelsträngiger Bereich a zwischen Oligonukleotid P2 und Oligonukleotid P3 aufgefüllt wird, bevor die Transkription durchgeführt wird.

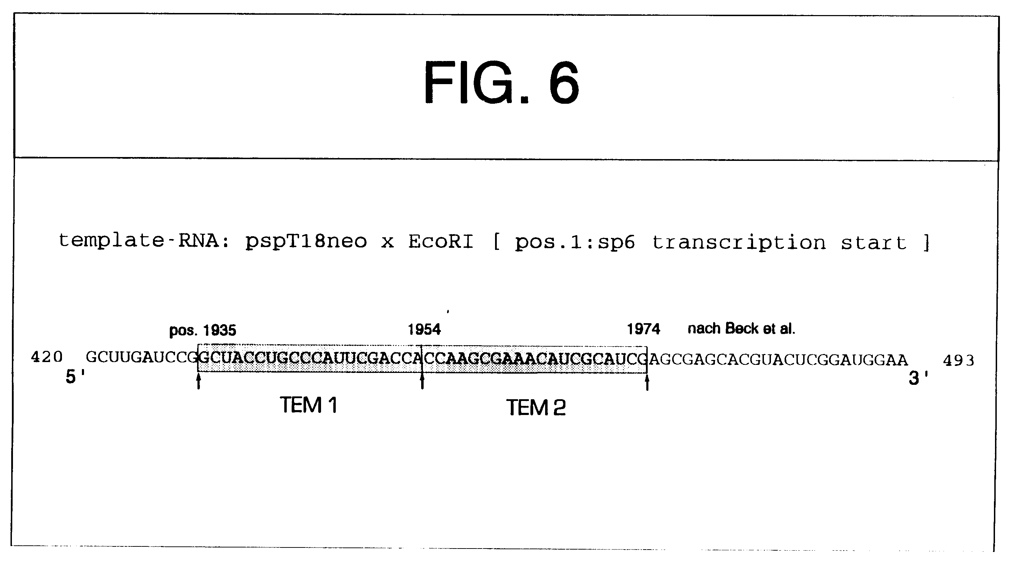

In Figur 6 ist der relevante Teil einer Templatnukleinsäure wiedergegeben. Es handelt sich um das Plasmid pSPT18neoxEco R1. Die Regionen, in welchen Oligonukleotid P2 und Oligonukleotid P3 anhybridisieren können, sind angegeben.

In Figur 7 und 8 sind die Nukleotidsequenzen für Oligonukleotide P1, P2 und P3 für zwei verschiedene Ausführungsformen angegeben.

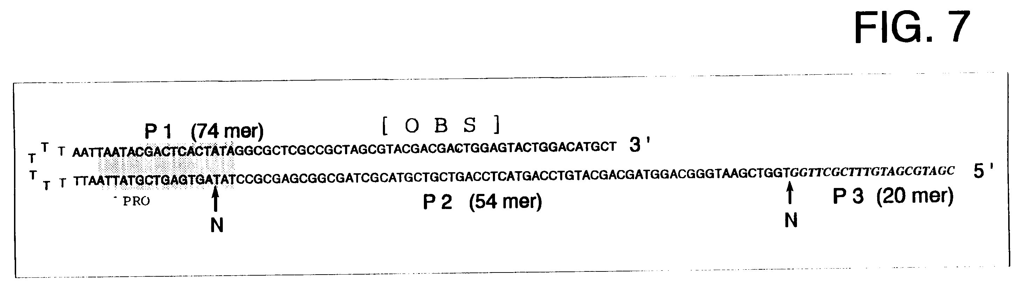

In Figur 7 ist der Fall gezeigt, daß es sich bei P1 um ein Haarnadel-Oligonukleotid mit 74 Nukleotiden handelt, wobei das 5'-Ende von P1 drei Nukleotide vom Transkriptionsstartpunkt entfernt innerhalb der Sequenz PRO liegt.

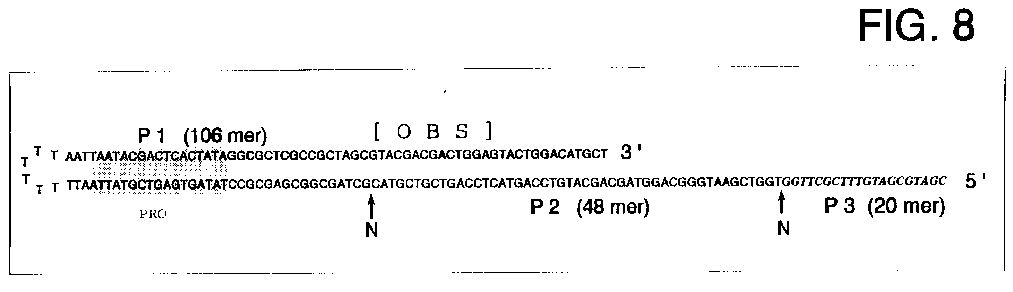

In Figur 8 ist der Fall beschrieben, wobei P1 106 Nukleotide umfaßt und das 5'-Ende 16 Nukleotide vom Transkriptionsstartpunkt entfernt ist und außerhalb der Sequenz PRO liegt.

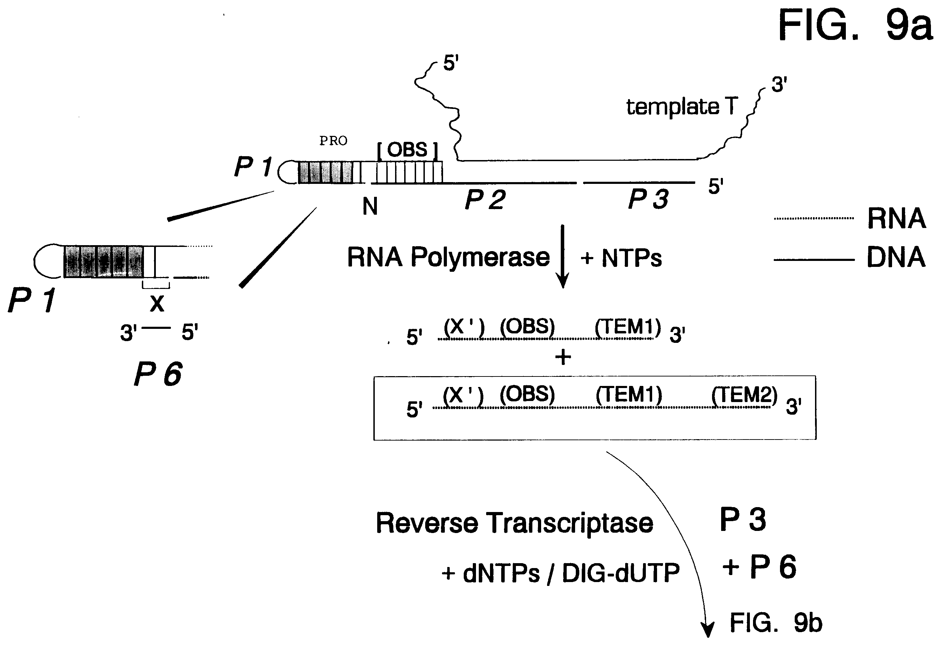

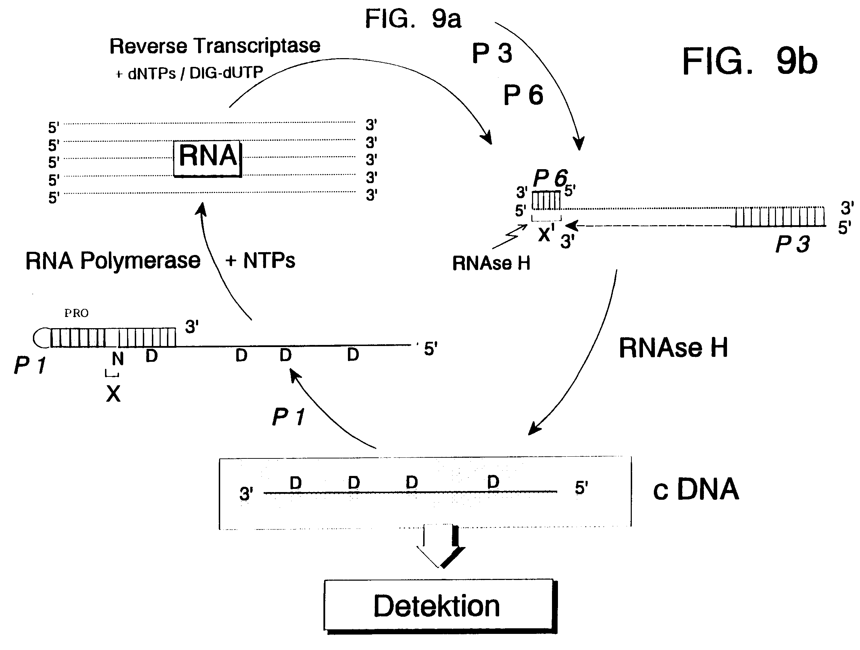

In Figur 9 (umfassend FIG 9a und 9b) ist die Verwendung der Oligonukleotide gemäß Figur 7 beschrieben, wobei jedoch zusätzlich ein Oligonukleotid P6 zugesetzt wird, das komplementär zu dem Bereich von P1 ist, der über den Transkriptionsstartpunkt hinausragt. Die Verwendung des Oligonukleotids P6 bewirkt, daß der mit X' bezeichnete Bereich der Transkripte nicht in cDNA umgeschrieben wird, so daß die cDNA ohne Überhang mit P1 hybridisieren kann.

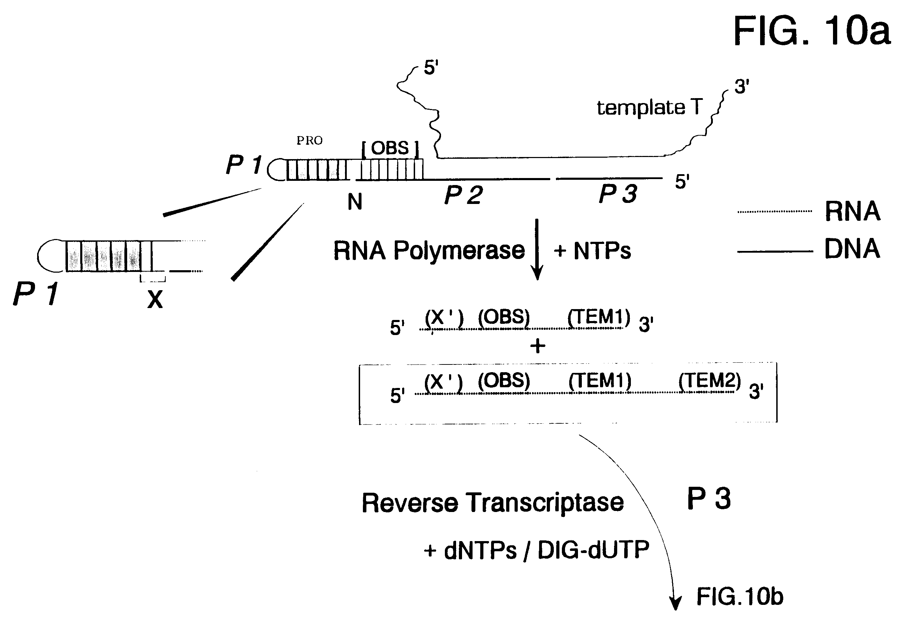

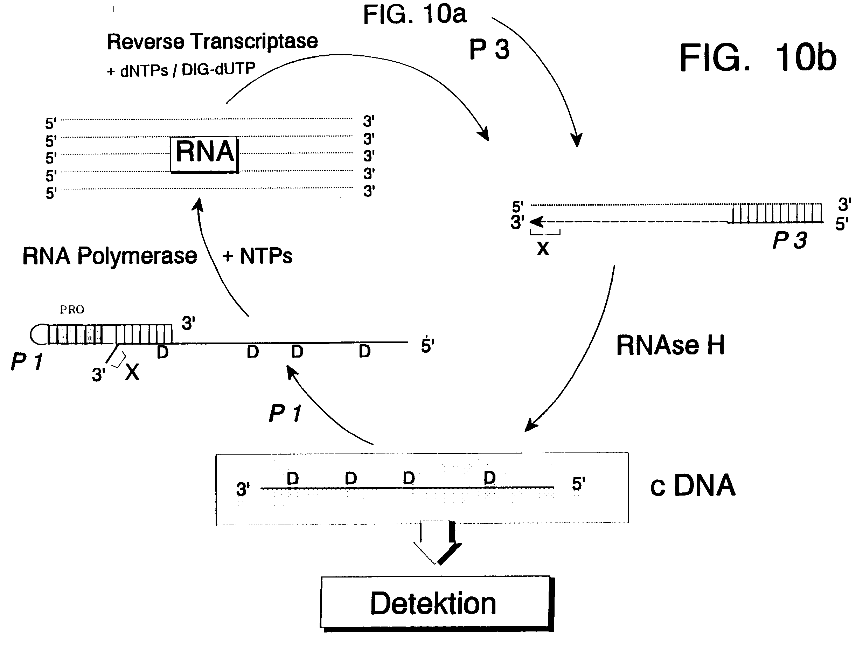

In Figur 10 (umfassend FIG 10a und FIG 10b) ist der Fall der Figur 8 beschrieben, wobei jedoch die Zugabe eines Oligonukleotids P6 unterlassen wurde. Der Überhang X der gebildeten cDNA stört jedoch die Transkriptionsreaktion nicht.

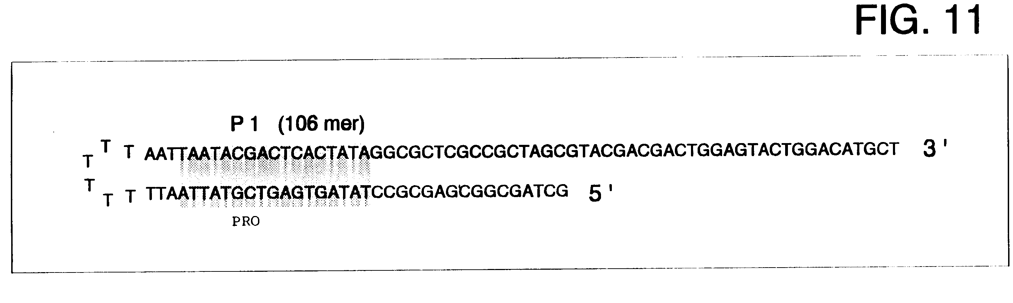

Figur 11 zeigt P1 des Beispiels 2.

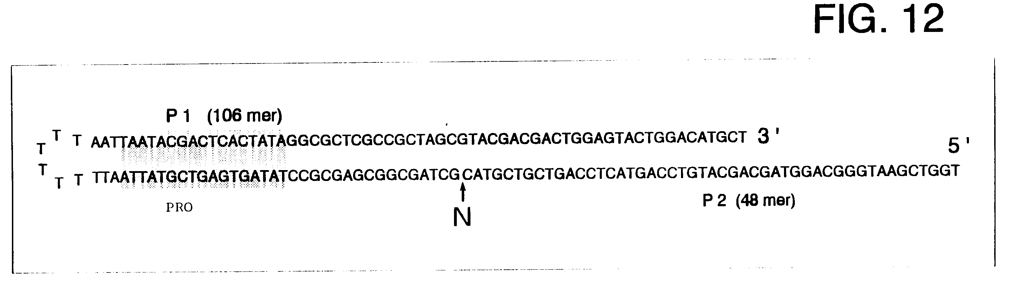

Figur 12 zeigt das Hybrid aus P1 und P2 aus Beispiel 3.

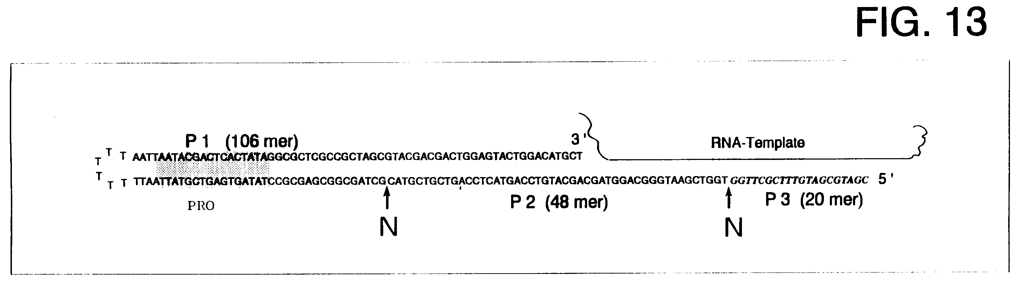

Figur 13 zeigt den transkribierbaren Komplex K aus Beispiel 4.

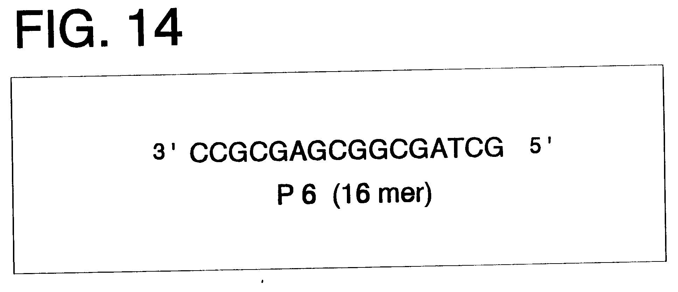

Figur 14 zeigt die Nukleotidsequenz von P6 aus Beispiel 5.

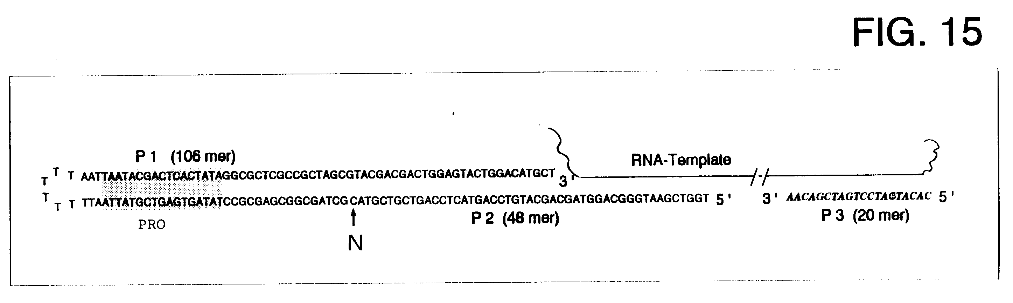

Figur 15 zeigt den transkribierbaren Komplex K für den Fall, daß das 3'-Ende von P3 vom 5'-Ende von P2 durch ein gap getrennt ist.

### Bezugszeichenliste

- P: templatspezifisches Promotorreagenz
- T: Templatnukleinsäure
- K: transkribierbarer Nukleinsäurekomplex aus P und T bzw. P, P3 und T
- K': transkribierbarer Nukleinsäurekomplex aus P1 und P2 (Nebenprodukt)
- L: transkribierbarer Komplex aus P1 und G
- R: Transkript (RNA)
- P1: Promotoroligonukleotid
- P2: templatspezifisches Oligonukleotid
- P3: Primeroligonukleotid
- P4: Oligonukleotid komplementär zu TEM 3
- P5: Oligonukleotid komplementär zu TEM 4
- P6: Oligonukleotid komplementär zu X
- PRO: Promotorbereich (doppelsträngig)
- OBS: Oligonukleotid-P2-Bindungsstelle
- OBS': mit der gesamten OBS hybridisierbare Sequenz von P2
- TEM 1: proximal zum Transkriptionsstartpunkt auf der Templatnukleinsäure gelegener Bereich
- TEM 2: distal zum Transkriptionsstartpunkt auf der Templatnukleinsäure gelegener Bereich
- TEM 1': templatspezifischer Bereich von P2
- TEM 2': templatspezifischer Bereich des Oligonukleotids P3
- TEM 3: Nukleotidbereich upstream von TEM 1
- TEM 4: Nukleotidbereich downstream von TEM 2
- X: Nukleotidsequenzen downstream des Transkriptionsstartpunktes
- X': zu X komplementäre Sequenzen
- G: Gegenstrang
- RT: Reverse Transkriptase
- BIO: Biotin
- D bzw.: DIG Digoxigenin
- NTP: Ribonukleosidtriphosphat
- dNTP: Desoxyribonukleosidtriphosphat
- a: Auffüllbereich
- a': Sequenz komplementär zum Auffüllbereich
- N: Einzelstrangbruch (Nick)

Die folgenden Beispiele erläutern die Erfindung näher.

### Beispiel 1

### Herstellung von RNA-Templates

Das Plasmid pSPT18 (Sequenz vgl. WO 89/06698) wird zur Herstellung von Transkripten des Neomycin-Resistenzgens (neo) verwendet. In pSPT18 wird das Neomycin-Gen (eine Aminoglycosid-3'-phosphotransferase II) wie in Beck et al. (1982), Gene, 19, 327 - 336, beschrieben, inseriert. Über das resultierende Plasmid pSPT18neo können mit SP6 RNA-Polymerase Transkripte des Gens in der Sense-Orientierung produziert werden. Plasmid pSPT18neo wird mit der Restriktionsendonuklease EcoRI linearisiert. Aus diesem linearisierten Plasmid werden durch in vitro Transkription, wie in Biochemicals for Molecular Biology, Boehringer Mannheim (1990), Seite 158, beschrieben, 1028 Nukleotide lange RNA-Transkripte hergestellt. Position 1 des Transkripts bezeichnet den SP6 RNA Polymerase Transkriptionsstartpunkt bzw. das erste Nukleotid des Transkripts. Nach Phenol-Extraktion zur Abreinigung enzymatischer Komponenten und Ethanol-Fällung können die so gereinigten Transkripte in den folgenden Beispielen als Templatnukleinsäuren eingesetzt werden.

### Beispiel 2

### Transkription an Primer P1 (Promotorkonstrukt mit loop-Struktur)

### a) Herstellung des Promotor-Primers P1

P1 besteht aus einem Nukleinsäurestrang von 106 nt, wobei 28 nt an dessen 3'- Ende der komplementären Sequenz am 3'-Ende von P2 entsprechen. Zusätzlich enthält P1 die minimal notwendige selbst-komplementäre Sequenz des Promotors für die RNA-Polymerase des Bakteriophagen T7 (Sequenz vgl. P1 in Fig. 12) (J.F. Milligan et al. (1987), Nucl. Acids Res., Vol. 15, No. 21, 8783-8798). Diese selbstkomplementären Sequenzen sind durch eine AT-reiche Region voneinander getrennt, die die Ausbildung des partiellen Doppelstrangs in Lösung fördert. Außerdem enthält der P1 zusätzliche, die Transkription fördernde, wie bei J.F. Milligan et al. (1987), Nucl. Acids Res., 15, S. 8783 - 8798 beschrieben, selbst-komplementäre aber nicht Template- komplementäre transkribierfähige Sequenzen am 5'-Ende downstream des Promotors.

Das dem P1 entsprechende DNA Oligonukleotid von 106 Nukleotiden wird nach der Synthese in einem DNA Synthesizer über ein 20%iges denaturierendes Polyacrylamid-Gel durch Elektrophorese, wie in Molecular Cloning (1989), Editors Sambrook, Fritsch, Maniatis, CSH, Seiten 6.39 - 6.48 beschrieben, gereinigt. Um ein möglichst vollständiges Annealing der selbstkomplementären Sequenzen in P1 zu ermöglichen, wird dieses DNA Oligonukleotid nach der Reinigung in einem Reaktionsgefäß 10 Minuten auf 90°C erhitzt und anschließend 10 Minuten auf Eis abgekühlt. P1 wird auf seine Fähigkeit, in Gegenwart von T7 RNA Polymerase unter den nachfolgend beschriebenen Versuchsbedingungen RNA-Transkripte zu produzieren, untersucht.

### b) Transkriptionsreaktion

Das Reaktionsgemisch enthält in einem Endvolumen von 25 µl:
40 mmol/l Tris-HCl (pH 8.0 bei 37°C), 6 mmol/l MgCl₂, 10 mmol/l NaCl, 10 mmol/l Dithiothreitol (DTT), 2 mmol/l Spermidin-HCl, 5 % (v/v) Polyethylenglycol MW 6000, 0,01 % (v/v)Triton-X-100, je 2 mmol/l ATP, UTP, GTP, CTP (pH 8.0 bei 37°C), 5 µci [ 32P]-CTP (400 Ci/mmol, Amersham), 500 nmol/l Primer 1, 15 U/µl T7-RNA-Polymerase (Boehringer Mannheim), 1 U/µl RNAse Inhibitor (Boehringer Mannheim)
Die nicht enzymatischen Einsatzstoffe werden vor der Verwendung mit 0,01% Diethylpyrocarbonat, wie in Molecular Cloning (s.o.) Seiten 7.3 - 7.4 beschrieben, behandelt.

In einem Reaktionsgefäß von 100 µl Inhalt werden die einzelnen Komponenten gemischt und der Ansatz eine Stunde bei 37°C inkubiert.

### c) Detektion

Anschließend wird die Reaktion durch Zugabe des gleichem Volumens Formamid-Stoppuffer (95% Formamid, 25 mM EDTA, 0,01% Xylencyanol, 0,01% Bromphenolblau), 3 minütiges Erhitzen auf 68°C und Abkühlung des Reaktionsansatzes auf Eis gestoppt. Ein Aliquot des denaturierten Reaktionsansatzes wird dann auf ein 7 M Harnstoff, 12% Polyacrylamid-Gel einer Schichtdicke von 0,8 mm aufgetragen. Die Gelelektrophorese wird analog U.K. Laemmli (1970), Nature, 277, S. 680-685 durchgeführt. Das Gel wird anschließend autoradiographiert und die radioaktiven Produkte analysiert.

Die Reaktion kann ebenfalls durch Zugabe von 10 mmol/l EDTA und 0,1% SDS gestoppt werden. Zur Detektion werden die Transkriptionsprodukte dann, wie in Molecular Cloning (s.o.), Seiten 7.43 - 7.45 beschrieben, in einem 1,5%igem denaturierendem Agarosegel aufgetrennt und die Reaktionsprodukte durch Färbung in einer Acridiniumorange-Lösung (5 µg/ml) sichtbar gemacht.

Wird dem Reaktionsansatz kein [ 32P]-CTP zugegeben, können die spezifischen Reaktionsprodukte, nach Gelelektrophorese in Polyacrylamid-Gelen über Northern-Blotting auf eine Nylonmembran transferiert und UV-fixiert, durch in-situ Hybridisierung mit komplementären, radioaktiv oder nichtradioaktiv markierten (Biochemicals for Molecular Biology, s.o., S. 112 - 115) DNA Oligonukleotiden detektiert werden. Diese Art von Hybridisierungen sind bei J. Meinkoth and G. Wahl (1984), Anal.Biochem., 138, S. 267-284, und in "Nucleic Acid Hybridisation" (1985) Editors B.D. Hames and S.J. Higgins, IRL Press, Oxford, S. 139 -159 beschrieben.

Ebenfalls können die Reaktionsprodukte, nach Gelfiltration mit einer Sephadex G-50 Säule von nichteingebautem [ 32P]-CTP getrennt, durch Ethanol-Fällung konzentriert und durch Auftropfen auf eine Nylonmembran, UV-Fixierung und Exposition eines Röntgenfilms mit der getrockneten Membran über die resultierende Schwärzung des Films detektiert werden.

Durch den Einbau von nichtradioaktiv markierten NTPs statt [ 32P]-CTP können die Produkte im DOT-, SLOT- oder Northern-blot direkt sichtbar gemacht werden. Der Einbau von Digoxigenin-11-UTP bzw. Biotin-16-UTP (vgl. WO 89/06698) kann zum direkten Nachweis mit Anti-Digoxigenin-AP-Konjugat bzw. mit Streptavidin-AP-Konjugat verwendet werden.

Die Detektion wird durch Reaktion von Alkalischer Phosphatase (AP) mit dem entsprechenden Substrat 5-bromo-4-chloro-3-indolyl-phosphat (X-Phosphat) und nitrobluetetrazolium salt (NBT), durch Farbumschlag der Reaktionslösung wie in Biochemicals for Molecular Biology (s.o.) S. 109 - 115 beschrieben, oder über eine durch Alkalische Phosphatase vermittelte Chemilumineszenzreaktion mit 3-(2'Spiroadamantan)-4-methoxy-4-(3''- phosphoryloxy)-phenyl-1,2-dioxetan (AMPPD^{R}, Boehringer Mannheim), wie bei I. Bronstein and P. McGrath (1989), Nature, 338, S. 599 - 600, beschrieben, ermöglicht.

Die Sensitivität der Chemilumineszenzreraktion kann durch Zusatz von 5,6 umol/l 5-N-tetradecanoylaminofluorescein (Fluoreszenzenhancer) in 0,75 mol/l 2-amino-2-methyl-1-propanol-Puffer, pH 9,6, wie bei M. Musani et al. (1991) Anal.Biochem., 194, S. 394 - 398 beschrieben, noch erhöht werden. Die Dokumentation dieser Lichtemission durch Chemilumineszenz erfolgt über die Belichtung eines Polaroid- oder Röntgenfilms.

Es wird RNA in der Länge vom Transkriptionsstart am Promotor bis zum 5'-Ende des Oligonukleotids P1 produziert.

### Beispiel 3

### Transkription des Hybrids aus P1 und P2

### a) Herstellung des P2

P2 besteht aus einem Nukleinsäurestrang von 48 nt, wobei 28 nt an dessen 3'- Ende der komplementären Sequenz am 3'-Ende P1 entsprechen (Sequenz vgl. Fig. 13). Zusätzlich enthält P2 am 5'-Ende 20 nt, welche komplementär zu den Ribonukleotidpositionen 431 - 450 des in Beispiel 1 beschriebenen RNA-Transkripts bzw. komplementär zu den Nukleotidpositionen 1935 - 1954 der Sequenz, welche nach Beck et al. das neo-Gen enthält (s.o.) sind. Das P2 entsprechende DNA Oligonukleotid von 48 Nukleotiden wird nach der Synthese, wie in Beispiel 2 beschrieben, gereinigt.

### b) Transkriptionsreaktion

Die Transkriptionsreaktion wird unter gleichen Bedingungen wie in Beispiel 2 durchgeführt.

Um ein möglichst vollständiges Annealing der komplementären Sequenzen der P1 und P2 zu ermöglichen, werden äquimolare Mengen dieser DNA Oligonukleotide vor der Zugabe der übrigen Reaktionskomponenten in einem Reaktionsgefäß 10 Minuten auf 90°C erhitzt und 10 Minuten auf Eis abgekühlt. Anschließend werden die denaturierten DNA Oligonukleotide 10 Minuten bei 37°C hybridisiert. Der Transkriptionspuffer und die enzymatischen Komponenten (s. Beispiel 2) werden in einem Reaktionsgefäß gemischt und die vorhybridisierten Oligonukleotide P1 und P2 in einer Endkonzentration von je 500 nmol/1 zugegeben. Der Reaktionsansatz wird eine Stunde bei 37°C inkubiert.

Anschließend werden die Reaktionsprodukte, wie in Beispiel 2 beschrieben, detektiert und die RNA-Produkte analysiert.

Es wird RNA in der Länge vom Transkriptionsstart am Promotor innerhalb P1 bis zum 5'-Ende von P2 produziert.

### Beispiel 4

### Transkription eines Hybrids aus P1, P2, P3 und RNA-template bzw. Oligonukleotid-template bzw. Plasmidtemplate

### a) Herstellung von P3 und des Oligonukleotid-template

Die Sequenz von P3 (DNA Oligonukleotid von 20 Nukleotiden; Sequenz vgl. Fig. 14) ist komplementär zu den Ribonukleotidpositionen 451 - 470 des in Beispiel 1 beschriebenen RNA-Transkripts bzw. zu den Nukleotidpositionen 1955 - 1974 der unter Beck et al. (s.o.) beschriebenen Sequenz, welche das neo-Gen umfaßt. Diese komplementäre Sequenz ist so gewählt, daß das 3'-Ende von P3 unmittelbar benachbart zum 5'-Ende von P2 mit dem RNA-Template hybridisieren kann. Das P3 entsprechende DNA Oligonukleotid von 20 Nukleotiden wird nach der Synthese, wie in Beispiel 2 beschrieben, gereinigt. Die Sequenz des Oligonukleotid-templates (DNA Oligonukleotid von 38 Nukleotiden) ist homolog zu den Ribonukleotidpositionen 431 - 468 des in Beispiel 1 beschriebenen RNA Transkripts. Diese zum RNA-Transkript homologe Sequenz ist so gewählt, daß die Templatekomplementären Bereiche von P2 und P3 direkt benachbart an das Oligonukleotid-Template hybridisieren.

### b) Transkriptionsreaktion

Die Transkriptionsreaktion wird wie in Beispielen 2 - 3 durchgeführt. Um ein möglichst vollständiges Annealing der komplementären Sequenzen von P1, P2, P3 und des RNA-Templates zu ermöglichen, werden diese DNA Oligonukleotide vor der Zugabe der übrigen Reaktionskomponenten in einem Reaktionsgefäß 10 Minuten auf 90°C erhitzt und 10 Minuten auf Eis abgekühlt. Durch diesen Denaturierungsschritt wird auch die Ausbildung möglicher Sekundärstrukturen innerhalb des RNA-Templates unterbunden. Anschließend werden die Komponenten 10 Minuten bei 37°C hybridisiert. Statt RNA-Template kann auch ein Oligonukleotid-Template, welches zumindest die neo-Sequenz von 1935 bis 1972 enthält, zur Hybridisierung mit P2 und P3 eingesetzt werden. Der Transkriptionspuffer und die enzymatischen Komponenten (s. Beispiel 2) werden in einem Reaktionsgefäß gemischt und die vorhybridisierten Oligonukleotide P1, P3 und RNA-Template in einer Endkonzentration von je 500 nmol/l und P2 in einer Endkonzentration von 50 nmol/l zugegeben. Der Reaktionsansatz wird eine Stunde bei 37°C inkubiert.

Anschließend werden die Reaktionsprodukte, wie in Beispiel 2 beschrieben, detektiert und die RNA-Produkte analysiert. Es wird RNA in der Länge vom Transkriptionsstart am Promotor innerhalb von P1 bis zum 5'-Ende des Primers 3 produziert.

### Beispiel 5

### Amplifikation durch Transkription eines Hybrids aus P1, P2, P3 und RNA-Template bzw. Oligonukleotid-Template unter Anwesenheit von P6, Reverser Transkriptase und RNAseH

### a) Herstellung von P6

Die Sequenz des P6 (DNA Oligonukleotid von 16 Nukleotiden; Sequenz vgl. Fig 15) ist homolog zu den Nukleotidpositionen 1 - 16 von P1. Diese homologe Sequenz ist so gewählt, daß sie vollständig mit dem in Beispiel 2 produzierten Transkript hybridisieren kann. In der nachfolgend beschriebenen Amplifikationsreaktion stellt dieses partielle RNA/DNA Hybrid ein Substrat für die RNaseH dar.

Das dem Primer 6 entsprechende DNA Oligonukleotid von 16 Nukleotiden wird nach der Synthese, wie in Beispiel 2 beschrieben, gereinigt.

Ebenso dient für die beschriebene Reaktion das Plasmid pSPT18neo, welches vor Hybridisierung mit P1, P2 und P3 auf 95°C erhitzt wurde.

### b) Amplifikationsreaktion

Die Amplifikationsreaktion wird wie die Transkriptionsreaktion in den Beispielen 2 - 4 mit folgenden Änderungen durchgeführt: Der Reaktionsansatz enthält jedoch keinen RNAse Inhibitor und zusätzlich werden dATP, dCTP, dGTP und dTTP in einer Konzentration von 1 mmol/l, AMV Reverse Transkriptase (Boehringer Mannheim GmbH) in einer Konzentration von 0,4 U/µl und RNAseH (Boehringer Mannheim GmbH) in einer Konzentration von 0,005 U/µl zugegeben.

Um ein möglichst vollständiges Annealing der komplementären Sequenzen der P1, P2, P3 und des RNA-Templates bzw. das Oligonukleotidtemplate zu ermöglichen, werden sie vor der Zugabe der übrigen Komponenten, wie in den Beispielen 2 - 4 beschrieben, denaturiert. Der Transkriptionspuffer und die enzymatischen Komponenten werden in einem Reaktionsgefäß gemischt und die denaturierten Oligonukleotide P1, P3 und P6 in einer Endkonzentration von je 500 nmol/l, Primer 2 in einer Endkonzentration von 50 nmol/l und das RNA-Template bzw. das Oligonukleotid-Template in einer Endkonzentration von 100 - 10⁶ Molekülen pro Reaktionsansatz zugegeben. Der Reaktionsansatz wird zwei Stunden bei 37°C inkubiert.

Anschließend werden die Reaktionsprodukte, wie in Beispiel 2 beschrieben, detektiert und die RNA-Produkte analysiert.

Es kann RNA in der Länge vom 3'-Ende von P2 bis zum 5'-Ende von P3 detektiert werden.

### Beispiel 6

Amplifikation durch Transkription eines Hybrids aus P1, P2, P3, und RNA-Template bzw. Oligonukleotid-Template unter Anwesenheit von P6, Reverser Transkriptase, und RNAseH, Markierung der cDNA während der Synthese durch Einbau von Digoxigenin-11-dUTP und anschließender Separation der spezifischen cDNA-Reaktionsprodukte über biotinmarkiertes komplementäres Oligonukleotid an eine Festphase.

### a) Herstellung des biotinmarkierten Oligonukleotids

Die Sequenz des biotinmarkierten Oligonukleotids mit einer Länge von 27 Nukleotiden ist komplementär zu den Nukleotidpositionen 1 - 10 von P2 und den Nukleotidpositionen 11 - 20 von P3. Die übrigen 7 Nukleotide sind nicht komplementär zu Sequenzen des RNA-Templates. Die Sequenz ist so gewählt, daß sie unter den nachfolgend beschriebenen Hybridisierungsbedingungen nur mit reaktionsspezifischen Produkten hybridisieren kann. Die Biotinmarkierung des Oligonukleotids ist in EP-A-0097373 beschrieben.

### b) Amplifikationsreaktion

Die Amplifikationsreaktion wird, wie in Beispiel 5 beschrieben, mit folgenden Änderungen durchgeführt: Der Reaktionsansatz enthält jedoch kein [ 32P]-CTP. Die dTTP Konzentration beträgt 0,66 mmol/l und es wird zusätzlich Digoxigenin-11-2'-desoxy-uridin-5'-dUTP (DIG-[11]-dUTP, Boehringer Mannheim) in einer Endkonzentration von 0,33 mmol/l zugegeben (vgl. WO 89/06698).

### c) Detektion

Nach der Amplifikationsreaktion werden die Produkte, wie in Beispiel 2 beschrieben, durch Gelfiltration mit Sephadex G-50 von nicht eingebauten Nukleotiden getrennt. Anschließend werden die Produkte durch 10 minütige Inkubation bei 92°C denaturiert und 4 Stunden mit 20 ng des biotinmarkierten Oligonukleotids in 200 µl mit 10% Formamid, 5x SSC, 1x Denhardt'sche Lösung und 50 mmol/l Natriumphosphat, pH 6.8, in einer Kavität einer mit Streptavidin beschichteten Mikrotiterplatte (hergestellt nach EP-A-0 344 578) bei 45°C inkubiert. Dabei beträgt die Endkonzentration des biotinmarkierten Oligonukleotids 8 nmol/l. Nach Hybridisierung / Wandbindung wird zweimal 10 Minuten bei 37°C mit 200µl 0,3 mol/l NaCl, 0,003 mol/l Na-Citrat, 0,2% SDS und einmal kurz bei Raumtemperatur mit 0,9% NaCl gewaschen. Nach der Zugabe von 200 mU/ml anti-Digoxigenin-Antikörper-Meerrettichperoxidase- Konjugat in 100 mmol/l Tris-HCl (pH 7,5), 0,9% NaCl, 1% RSA wird bei 37°C unter Schütteln 30 Minuten inkubiert. Nicht gebundenes Konjugat wird durch 3-maliges Waschen mit 0,9 % NaCl bei Raumtemperatur entfernt. Nach 30 Minuten Inkubation bei 37°C mit 1,9 mmol/l 2,2'-Azino-di-[3-ethylbenzthiazolin-sulfonsäure]-(6)-diammoniumsalz (ABTS^{R} , Boehringer Mannnheim) wird die Extinktion bei 405 nm mittels eines ELISA-Readers gemessen.

Die Detektion kann, wie in Beispiel 2 beschrieben, aber hier nach dem Einbau von Digoxigenin-11-dUTP bzw. Biotin-16-dUTP während der cDNA Synthese, nachfolgendem Southern-Blot auf eine Nylon-Membran und anschließender UV-Fixierung, direkt über den Nachweis mit Anti-Digoxigenin-AP-Konjugat bzw. Streptavidin-AP-Konjugat erfolgen.

### Beispiel 7

### Amplifikation durch Transkription eines Hybrids aus P1, P2, P3 und RNA-Template unter Anwesenheit von P6, Reverser Transkriptase und RNAseH

### a) Herstellung von P3

Die Sequenz von P3 (DNA Oligonukleotid von 20 Nukleotiden; Sequenz vgl. Fig. 16) ist komplementär zu den Ribonukleotidpositionen 501 - 520 des in Beispiel 1 beschriebenen RNA-Transkripts bzw. zu den Nukleotidpositionen 2005 - 2024 der unter Beck et al. (s.o.) beschriebenen Sequenz, welche das neo-Gen umfaßt. Diese komplementäre Sequenz ist so gewählt, daß das 3'-Ende von P3 50 Nukleotide entfernt zum 5'-Ende des Primers 2 mit dem in Beispiel 1 beschriebenen RNA-Template hybridisieren kann. Das P3 entsprechende DNA Oligonukleotid von 20 Nukleotiden wird nach der Synthese gereinigt (s. Beispiel 2).

### b) Amplifikationsreaktion

Die Amplifikationsreaktion und die Detektion wird, wie in Beispiel 5 oder in Beispiel 6 beschrieben, mit folgender Änderung durchgeführt: Der Reaktionsansatz enthät P1, P3, P6 in einer Endkonzentration von 500 nmol/l und P2 in einer Endkonzentration von 50 nmol/l.

Das bei der Amplifikationsreaktion synthetisierte RNA-Produkt bzw. cDNA-Produkt kann auch spezifisch über die Hybridisierung mit einem komplementären biotinmarkierten Oligonukleotid, wie in Beispiel 2 und 6 beschrieben, detektiert werden. Dabei wird die Sequenz dieses biotinmarkierten Oligonukleotids so gewählt, daß seine hybridisierfähige Region komplementär zu dem Bereich der RNA bzw. cDNA ist, die während der Amplifikation zwischen P2 und P3 synthetisiert wird.

Es kann RNA, wie in Beispiel 5 beschrieben, bzw. cDNA, wie in Beispiel 6 beschrieben, in der Länge vom 3'-Ende von P2 bis zum 5'-Ende von P3 detektiert werden.

### Beispiel 8

### Bandbreite der Reaktion

Im Folgenden werden Reaktionsbedingungen angegeben, innerhalb derer das erfindungsgemäße Verfahren abläuft. Der Fachmann kann jedoch damit aufgrund weniger Versuche auch hiervon abweichende Bedingungen ermitteln.

| | optimale Bedingungen | Bandbreite |
|---|---|---|
| Tris-HCl | 40 mM (pH 8.0) | 2 - 150 mM (pH 7.5 - 8.5) |
| MgCl2 | 6 mM | 2 - 20 mM |
| NaCl | 10 mM | 0 - 200 mM |
| DTT | 10 mM | 2 - 20 mM |
| Spermidin-HCl | 2 mM | 0 - 10 mM |
| PEG 6000 | 5 % | 0 - 10 % |
| Triton-X-100 | 0.01 % | 0 - 0.5 % |
| USA | - | 0 - 100 µg/ml |
| RNAse Inhibitor | 1 U/µl | 0 - 5 U/µl |
| | | |
| dNTPs | 1 mM | 0.1 - 5 mM |
| NTPs | 2 mM | 0.2 - 5 mM |
| | | |
| Primer 1/3/6 | 500 nM | 100 nM - 1.5 µM |
| Primer 2 | 50 nM | 1 - 500 nM |
| | | |
| RNA - Polymerasen | T7 RNA Polymerasen | T7, SP6, T3, N4, RNA Polymerasen |
| T7 PNA Polymerasen | 15 U/µl | 5 - 25 U/µl |
| Reverse Transkriptase | AMV Reverse Transkriptase | AMV oder Mo-MLV Reverse Transkriptase |
| Reverse Transkriptase | 0.4 U/µl | 0.2 - 10 U/µl |
| RNAse H | 0.005 U/µl | 0.001 - 0.05 U/µl |
| | | |
| Reaktionstemperatur | 37°C | 35 - 42°C |
| Reaktionszeit | 60 min. | 20 min. - 3 Std. |
| Reaktionsvolumen | 25 µl | 20 - 200 µl |
| Vorhybridisierung | 0-30 min | 0-6 h |

## Patentansprüche

1. Verfahren zur spezifischen Herstellung von Nukleinsäuren durch
- Umsetzung eines Promotorreagenzes P mit einer Templatnukleinsäure T unter Bildung eines transkribierbaren Nukleinsäurekomplexes K und
- promotorgesteuerte Transkription unter Bildung von Transkripten R,
dadurch gekennzeichnet, daß das Promotorreagenz P ein Promotoroligonukleotid P1 und ein damit hybridisierbares templatspezifisches Oligonukleotid P2 enthält.

2. Verfahren zum spezifischen Nachweis von Nukleinsäuren durch
- Umsetzung eines Promotorreagenzes P mit einer Templatnukleinsäure T unter Bildung eines transkribierbaren Nukleinsäurekomplexes K,
- promotorgesteuerte Transkription unter Bildung von Transkripten R und
- Nachweis der Transkripte R oder deren Folgeprodukte,
dadurch gekennzeichet, daß das Promotorreagenz P ein Promotoroligonukleotid P1 und ein damit hybridisierbares templatspezifisches Oligonukleotid P2 enthält.

3. Verfahren gemäß Anspruch 1 oder 2, dadurch gekennzeichnet, daß der zahlenmäßige Anteil P2 in P geringer ist als der zahlenmäßige Anteil von P1 in P.

4. Verfahren gemäß Anspruch 3 dadurch gekennzeichet, daß
- zu den Transkripten R Gegenstränge G gebildet werden,
- diese mit P1 zu Hybriden H hybridisiert werden und
- diese Hybride erneut zur Bildung von Transkripten R verwendet werden.

5. Verfahren nach Anspruch 4, dadurch gekennzeichnet, daß als Enzym zur Transkription eine Polymerase verwendet wird, die Einzelstrangbrüche überliest.

6. Verfahren gemäß Anspruch 1, 2 oder 4, dadurch gekennzeichnet, daß der Nukleinsäurekomplex K benachbart zu dem Oligonukleotid P2 mindestens ein weiteres templatspezifisches Oligonukleotid P3 enthält.

7. Verfahren gemäß Anspruch 6, dadurch gekennzeichnet, daß der zahlenmäßige Anteil von P3 ungefähr dem Anteil von P1 entspricht.

8. Verfahren nach Anspruch 6, dadurch gekennzeichnet, daß die Oligonukleotide P1 und P2 in keiner Phase des Verfahrens miteinander ligiert werden.

9. Verfahren nach Anspruch 6, dadurch gekennzeichnet, daß die Oligonukleotide P2 und P3 in keiner Phase des Verfahrens miteinander ligiert werden.

10. Verfahren gemäß Anspruch 6, dadurch gekennzeichnet, daß der transkribierbare Komplex K neben den Oligonukleotiden P1, P2 und P3 mindestens ein einzelstrangstabilisierendes Oligonukleotid enthält, welches an einen Bereich anhybridisiert ist, der zu den Bereichen in denen P2 und P3 anhybridisiert sind, benachbart gelegen ist.

11. Verfahren gemäß Anspruch 6, dadurch gekennzeichnet, daß die Oligonukleotide P1 und P2 sowie P2 und P3 im Nukleinsäurekomplex K nur durch einen nick getrennt sind.

12. Reagenz zur Herstellung von Nukleinsäuren aus einer Templatnukleinsäure, enthaltend
- ein Promotoroligonukleotid P1, welches außer einem doppelsträngigen Promotorbereich PRO am 3'-Ende eine Oligonukleotidbindesequenz OBS enthält, und
- ein templatspezifisches Oligonukleotid P2, welches außer einem templatspezifischen Bereich TEM 1' einen mit OBS hybridisierbaren Bereich OBS' aufweist.

13. Reagenz gemäß Anspruch 12, dadurch gekennzeichnet, daß es außerdem ein Primer P3 enthält, der einen zweiten templatspezifischen Bereich TEM 2' aufweist.

14. Reagenzkit zur Herstellung von Nukleinsäuren, enthaltend in getrennten Behältern
- das Reagenz nach Anspruch 12 und Monoribonukleosidtriphosphate
- eine zum Promotor passende RNA-Polymerase.

15. Reagenzkit gemäß Anspruch 14 mit weiteren Behältern, enthaltend
- eine reverse Transkriptase;
- Monodesoxyribonukleosidtriphosphate; und
- ein RNA verdauendes Enzym.
